# EUROPEAN PATENT APPLICATION

(11) **EP 2 700 409 A1**
(43) Date of publication of application: **26.02.2014**
(21) Application number: 12181535.1
(22) Date of filing: 23.08.2012
(51) Int. Cl.: A61K 38/20, A61K 31/7088, A61K 39/395, A61P 11/00, A61P 31/16

(54) **IL-27 for modulation of immune response in acute lung disease**

(71) Applicant: Deutsches Rheuma-Forschungszentrum Berlin, 10117 Berlin (DE)
(72) Inventor: Witzenrath, Martin, 10781 Berlin (DE); Hamann, Alf, 15732 Eichwalde (DE); Liu, Francesca Diane, 13351 Berlin (DE)
(74) Representative: Lange, Sven

(57) **Abstract**

The invention relates to an interleukin-27 (IL-27) modulator for the treatment of a pathological immune response associated with acute lung disease. The invention therefore relates to IL-27 agonists, preferably IL-27 protein, for the treatment of immunopathology and/or uncontrolled inflammatory lung disease, as well as IL-27 antagonists for the treatment of immune response depression. The IL-27 agonist is preferably administered at a defined, preferably delayed, stage of infection or inflammation.

## Description

The invention relates to an interleukin-27 (IL-27) modulator for the treatment of a pathological immune response associated with acute lung disease. The invention therefore relates to IL-27 agonists, preferably IL-27 protein, for the treatment of immunopathology and/or uncontrolled inflammatory lung disease, as well as IL-27 antagonists for the treatment of immune response depression. The IL-27 agonist is preferably administered at a defined, preferably delayed, stage of infection or inflammation.

Infection with highly pathogenic influenza virus strains is associated with massive pulmonary infiltration and immunopathology. Using IL-27ra-/- mice, the role and mode of action of IL-27 in controlling excessive inflammation upon infection can be elucidated. Intact signalling through IL-27Rα is required for survival by limiting immunopathology and regulating IL-17 and IFN-γ production in T cells via IL-10-dependent and independent pathways. Consistent with its physiological role and timely expression after infection, systemic treatment with rIL-27 at different time-points during influenza has a surprisingly striking impact on immunopathology and viral control, whereby delayed treatment results in amelioration of lung pathology and improvement of disease course without affecting viral clearance. In contrast, early application led to increased virus load and worsened disease. These findings demonstrate the importance of well-timed production and/or administration of IL-27 for the physiological control of immunopathology and the value of IL-27 to treat life-threatening inflammation upon lung infection. In addition, IL-27 might be a cause of endogenous immunodepression or suppression, observed subsequent to certain infections such as influenza but also bacterial infections.

### BACKGROUND OF THE INVENTION

Interleukin 27 (IL-27) is a heterodimeric cytokine with complex functions in inflammation. IL-27 belongs to the IL-12 superfamily and is composed of the Epstein-Barr virus inducible gene-3 (EBI3) and the IL-27p28 subunit, which are related to IL-12p40 and IL-12p35 molecules, respectively (Pflanz et al., 2002). The IL-27 receptor complex consists of IL-27Rα (WSX-1, TCCR) and the gp130 subunit, and is expressed by a wide range of cell types including T and B cells, monocytes, mast cells and neutrophils (Hunter, 2005). Initially, IL-27 was thought to promote TH1 responses because of its ability to induce T-bet expression, thereby triggering the upregulation of IL-12β2 receptor (Hibbert et al., 2003). However, subsequent studies using in vivo models of infection and autoimmune diseases provided evidence of its function in limiting immune-mediated pathology (Stumhofer and Hunter, 2008). Mice deficient in IL-27 receptor developed overwhelming TH1 responses upon infection with a number of parasites and intracellular bacteria (Findlay et al., 2010; Hamano et al., 2003; Holscher et al., 2005; Rosas et al., 2006; Stumhofer et al., 2006; Villarino et al., 2003). These infected Il-27ra-/- mice exhibited uncontrolled IFN-γ responses by CD4+ T cells that were accompanied by increased immunopathology. Moreover, the lack of IL-27 receptor signaling resulted in augmented IL-17 production by CD4+ T cells in several animal models, including experimental autoimmune encephalomyelitis (EAE) (Amadi-Obi et al., 2007; Batten et al., 2006; Stumhofer et al., 2006). Most studies have characterized the ability of IL-27 to suppress CD4+ T cell responses, but the regulatory function of IL-27 also extends to cells of the innate immune system (Hunter, 2005). For example, Ebi3-/- mice exhibited resistance to experimental septic peritonitis, in part due to enhanced neutrophil migration and function, thereby leading to successful bacterial clearance (Wirtz et al., 2006).

Binding of IL-27 to its receptor induces a signaling cascade mediated by Janus kinases and activation of various STAT transcription factors including STAT1, STAT3, STAT4 and STAT5 in T cells (Hibbert et al., 2003; Lucas et al., 2003; Takeda et al., 2003; Villarino et al., 2003). Consistent with its regulatory function, IL-27-mediated activation of STAT1 and STAT3 promotes IL-10 and suppresses IL-17 production in CD4+ T cells (Anderson et al., 2009; Stumhofer et al., 2006; Yoshimura et al., 2006). IL-27 also induces the expression of the suppressor SOCS3 in CD4+ T cells, resulting in reduced IL-2 secretion in these cells (Owaki et al., 2005; Villarino et al., 2006).

Infection with highly pathogenic strains of influenza viruses, such as H5N1 and the pandemic 1918 Spanish flu, is characterized by rapid leukocytic infiltration of the lung and excessive production of pro-inflammatory cytokines including IFN-γ, TNF-α, and IL-6 (La Gruta et al., 2007; Peiris et al., 2010). Pathological findings strongly suggest that the vigorous mobilization of the innate and adaptive arms of host immunity upon infection plays a key role in mediating lung injury (Peiris et al., 2010).

The present invention is based upon IL-27 and its receptor IL-27Rα and their effect on the immune response to influenza virus. Influenza virus infected Il-27 Rα -/- mice exhibit increased mortality associated with immunopathology. Moreover, infected Il-27 Rα -/- mice exhibited augmented numbers of IFN-γ and IL-17-producing CD4+ and CD8+ T cells in the infected respiratory tract. Although IL-10 production by CD4+ T cells is decreased in infected Il27 Rα -/- mice, IL-10 was not necessary for the suppression of IFN-γ in T cells as influenza virus infected Il-10-/- mice have WT frequencies of IFN-γ. Further in vitro experiments show that the regulation of IFN-γ production by CD8+ T cells is directly mediated by IL-27Rα signalling. Importantly, carefully timed treatment with rIL-27 improved lung injury and weight loss during infection. The invention demonstrates that IL-27 has a distinct role in controlling immunopathology and therefore is an important medical tool for immunomodulatory therapy of infection and other acute inflammatory processes.

### SUMMARY OF THE INVENTION

In light of the prior art the technical problem underlying the present invention is to provide means for modulating a pathological immune response in patients with acute lung disease.

This problem is solved by the features of the independent claims. Preferred embodiments of the present invention are provided by the dependent claims.

Therefore, an object of the invention is to provide an interleukin-27-modulator for the treatment of a pathological immune response associated with acute lung disease.

The invention therefore relates to IL-27 modulators, including both agonists and antagonists of IL-27, for the treatment of a pathological immune response itself, which is caused by an acute lung disease that subsequently leads to the undesired immune response. The acute nature of the lung disease that causes the pathological immune response to be treated is an important defining aspect of the invention. The prior art has disclosed the use of IL-27 in the treatment of unwanted immune responses in chronic or allergic lung diseases. However, it was at the time of filing the present invention unknown that IL-27 played an important role in unwanted immune responses that occurred due to acute lung disorders, whether said lung disorders are either infectious or non-infectious.

The finding of the inventors, that IL-27 function is important in immune responses associated with acute lung diseases (in particular that administration or activation of IL-27 can suppress an unwanted immune response associated with acute lung disease) is a novel and inventive development of the prior art. The use of IL-27, or an agonist of endogenous or exogenous IL-27, in the suppression of an unwanted, preferably inflammatory, pathological immune response associated with acute lung disease has been neither disclosed nor suggested in the prior art.

The accepted notion in the field was that IL-27 became an important factor in persistent inflammatory immune responses (immunopathology) in a patient, for example in cases of prolonged chronic lung disorders or allergic disorders. The modulation of IL-27 in acute lung disease-associated immune responses is a novel feature of the present invention.

The acute lung diseases of the present invention differentiate themselves from chronic or allergic lung diseases by their pathologic and pathogenic nature, short induction times and non-repetitive frequency or recurrence in patients. It was previously unknown that IL-27 played a role in such acute cases of lung disease.

The invention therefore relates to an IL-27 modulator, characterised in that the modulator is
a. an IL-27 agonist, such as
   i. IL-27 protein, preferably recombinant IL-27, comprising of complete recombinant cytokine IL-27 or a fragment thereof that maintains activity, for example one of the IL-27 chains, p28 or EBI3,
   ii. a nucleic acid that encodes IL-27 according to any one of the preceding claims, or encodes an IL-27 fragment, that after administration and expression of said nucleic acids leads to the local production of IL-27 protein, or
   iii. a bacterial or viral host organism that comprises a nucleic acid that encodes IL-27 or a functional fragment thereof, for example one of the IL-27 chains, p28 or EBI3, or
b. an IL-27 antagonist, such as
   i. antibodies that bind to IL27, to its subunits p28 and/or EBI3, antibodies directed against the IL-27 receptor or its subunits, IL27-receptor fusion proteins competing for soluble IL-27, or
   ii. antagonistic compounds blocking the receptors including inactive or inactivated variants of IL-27 or its subunits, or
   iii. a nucleic acid that encodes IL27-receptor fusion proteins or antagonistic compounds according to any one of the preceding claims, that after administration and expression of said nucleic acids leads to the local production of an IL-27 antagonist.

In a preferred embodiment the IL-27 modulator of the present invention is characterised in that the acute lung disease is a non-allergic and/or non-chronic lung disease.

In one agonist the IL-27 modulator of the present invention is characterised in that the pathological immune response is an inflammatory immune response, such as pneumonia.

In a preferred embodiment the IL-27 agonist of the present invention is characterised in that the inflammation is caused by an infectious agent, such as viral, bacterial, fungal or parasitic infectious agents and may include concurrent infections by one or more of these agents.

In one embodiment the IL-27 agonist of the present invention is characterised in that the infectious agent is
- an infection by influenza-, rhino-, corona-, herpes (such as cytomegalovirus (CMV) or varizella zoster virus (VZV)), respiratory syncytial or other viruses,
- an infection by Streptococcus, Staphylococcus, Pseudomonas, Haemophilus, Chlamydophila or other bacteria including Pneumocystis, such as Pneumocystis jiroveci pneumoniam, and/or
- an infection by Aspergillus, Candida or other mycosis.

In one embodiment the IL-27 agonist of the present invention is characterised in that administration is carried out at an optimal and safe time point defined by a diagnostic tests for immunological parameters. In the context of the present invention optimal and safe time points are those commonly known to a skilled practitioner, such as a medical expert, who can assess the condition of a patient and consider if the time point for administration is optimal. Examples of optimal time points are provided herein, in addition to the means that can be used for determining these time points.

In one embodiment the IL-27 agonist of the present invention is characterised in that treatment with IL-27 agonist occurs at a defined stage of infection, preferably a delayed stage, whereby the viral and/or bacterial load during progression of the infection has gone beyond the exponential phase of proliferation post infection, for example when the peak viral and/or bacterial load has been attained, or preferably has passed. The term delayed stage does not inherently represent delay after identification or diagnosis of the infection or condition. A patient may for example already exhibit a delayed stage of infection at the point of diagnosis, thereby requiring immediate treatment. The delayed stage (as a preferred embodiment) relates to delay after infection initiation, not initial diagnosis.

It was especially surprising that in the context of an acute lung disorder, the modulation of IL-27, in a preferred embodiment the administration of IL-27 itself, at a time point in which the infection has attained or passed its peak (for example with regard to in vivo multiplication), leads to a particularly advantageous opportunity for treatment.

In one embodiment the appropriate time point for application of an IL-27 agonist of the present invention is characterised in that the stage of infection during progression of the infection is determined by measurement of
- circulating virus and/or bacteria, or viral and/or bacterial components, such as protein, RNA and/or DNA.
- specific immune indicators, such as markers of acute inflammation, for example IL-6, IL-8 or C-reactive protein (CRP),
- markers for infection, such as Procalcitonin or Lipopolysaccharide (LPS) binding protein (LBP), and/or
- antibodies, preferably specific antibodies, more preferably antibodies specific to infection-related virus and/or bacteria, or viral and/or bacterial components, and/or T cells in the patient and/or T cells in the patient.

The relationships between circulating virus and/or bacteria (or viral and/or bacterial components), specific immune indicators, markers for infection, antibodies and/or T cells in the patient and the stage (progression) of infection are well understood by one in the art. The amount of bacteria or virus detected in a patient via known techniques is routinely used to assess infection stage. Similarly, the use of immune indicators, or markers for infection are common tools used for determining infection progression. Importantly, a skilled person has these tools at his disposal in order to assess infection progression, and in a preferred embodiment, to administer IL-27 (or agonist thereof) at an optimal and safe time point, preferably at a defined stage, preferably delayed stage in relation to infection initiation as described herein, of infection defined by declining viral or bacterial survival.

More specifically, the preferred time point for start of treatment is defined by the first signs of declining titers of viral or bacterial material or by respective alterations in surrogate markers such as Procalcitonin or LBP in blood circulation.

In another situation, the decline in bacterial load is anticipated from previous knowledge on the effects of antibiotics used in primary therapy as commonly known to a skilled practitioner, such as a medical expert.

In a further aspect of the invention the IL-27 agonist of the present invention is characterised in that the inflammation is caused by a non-infectious agent or treatment, including but not restricted to mechanical ventilation, trauma, transfusion related, disseminated coagulopathy, acid aspiration, idiopathic (Haman-Rich Syndrome) and any other sterile causes of ARDS (acute respiratory distress syndrome).

In one embodiment the IL-27 agonist of the present invention is characterised in that the pathological immune response in the lung is an acute respiratory distress syndrome (ARDS or ALI).

In one embodiment the IL-27 agonist of the present invention is characterised in that the treatment comprises of daily administration of IL-27 agonist for a duration of 2 to 14 days, preferably 4 to 12 days, more preferably 5 to 10 days.

A further aspect of the invention relates to an IL-27 antagonist for the treatment of a pathological immune response, whereby the immune response is immune response suppression.

In one embodiment the IL-27 antagonist of the present invention is characterised in that the suppressed immune response occurs during or subsequent to influenza infection, preferably in case of multiple infections by bacterial and/or viral agents, for example double infections of influenza and pneumococcus.

In one embodiment the IL-27 antagonist of the present invention is characterised in that the occurrence of a immunodepressed state, as a indication for treatment, is determined by diagnostic tests measuring appropriate markers of an immunodepression including but not restricted to IL-27 levels in blood or other body fluids, IL-10, HLA-DR expression on monocytes, LPS-induced cytokine (e.g. TNF) in monocytes, CD64 expression in granulocytes, or any other parameter defining a condition of endogenous suppression of the immune system as known by skilled medical expert for immune diagnostics.

A further aspect of the invention relates to a pharmaceutical composition comprising the IL-27 modulator, the IL-27 agonist or the IL-27 antagonist of claims with a pharmaceutically acceptable carrier substance.

Although the invention relates to the treatment of pathological immune responses caused by acute lung disease, the IL-27 modulator as described herein can be characterised in that the acute lung disease leads eventually to chronic impairment of lung function, such as fibrosis, if not treated.

In one embodiment the IL-27 agonist of the present invention is characterised in that the treatment comprises a single administration of the agonist in a depot form of application, whereby known methods of prolonged delivery using pharmaceutical preparations of distinct application routes allow for a prolonged bioavailability of the agonist after a single administration.

In one embodiment the IL-27 agonist of the present invention is characterised in that administration is carried out by intravenous (i.v.), intramuscular (i.m.), intraperitoneal (i.p.) or sub-cutaneous (s.c.) injection of IL-27 agonist, by inhalation (spray, powder, aerosol) for either a local effect and/or for the purpose of resorption and a systemic effect.

The invention therefore also relates to the use of an interleukin-27-modulator (agonist or antagonist) for the treatment of a pathological immune response associated with acute lung disease, in addition to corresponding methods of treatment comprising administration of an interleukin-27-modulator (agonist or antagonist) for the treatment of a pathological immune response associated with acute lung disease.

### DETAILED DESCRIPTION OF THE INVENTION

IL-27 was detected in 2002 (Pflanz et al., 2002. IL-27, a Heterodimeric Cytokine Composed of EBI3 and p28 Protein, Induces Proliferation of Naive CD4+ T Cells. Immunity 16: 779-790) and has been described for application in diverse types of disease, including treatment of inflammatory diseases in various organs.

One aspect of the present invention relates to an investigation of the contribution of the cytokine IL-27 and its receptor on the immune response in influenza infection. The present invention therefore relates to the use of IL-27 in the therapy of undesired immune reactions.
a) The inventors observed a significant role of natural IL-27 in limiting immunopathology by suppressing the production of inflammatory cytokines by T-cells and the recruitment of cells of the innate immune system, such as neutrophils into the inflamed tissue. In the absence of the IL-27 receptor, influenza virus infected mice displayed a strong increase in immunopathology resulting in reduced survival. Effects of lacking IL-27 receptor were much more significant than a lack of IL-10 in this system, even though part of the effect of IL-27 is mediated through its IL-10 inducing capacity.
b) The inventors therefore tested administration of recombinant IL-27 and observed that treatment with recombinant IL-27 at the later stages of influenza infection (e.g. starting at peak of inflammation) strongly ameliorates the accumulation of leukocytes and the resulting immunopathology of the lung, while having little effects on the antigen-specific T cell response. Most notably, virus clearance was not affected or even improved upon early rIL-27-treatment. Delayed treatment, at the peak of inflammation, was safe and positively influenced viral clearance, immunopathology of the lung, and overall survival.

These findings confirm that IL-27 displays a potent immunomodulatory activity and represents a novel, natural immunosuppressant with a unique selectivity. Under the given conditions, its action is predominantly directed towards innate immune mechanisms. This distinguishes it from other immunosuppressants with either very broad action such as corticosteroids, or T cell-directed suppressants such as cyclosporine A.

The inventors conclude that treatment with recombinant IL-27, administered either systemically or by local application (inhalation) represents a useful strategy to fight severe conditions of immunopathology, notably those caused by innate leukocytes such as macrophages or neutrophils. On the other hand, blocking IL-27 could be beneficial in cases of natural immunosuppression or immunodepression caused by high levels of endogenously produced IL-27.

The present invention relates to the treatment of a novel sub-group of patients, who were previously not easily treated using the means known in the art. The treatment of influenza is often complicated by associated unwanted immunopathology, and late in an infection there was previously no reliable method for reducing such immune-related inflammation. In fact, previous attempts to treat immunopathology during influenza using unspecific immunosuppressants like corticosteroids were found to worsen the disease. The present invention represents a novel medical use of IL-27, which provides unexpected advantages in the modulation of immune reactions. The patient groups comprising individuals with either too low or excessive immune responses have previously not been easily treated, whereby the IL-27 application in these patient groups enables modulation of the unwanted or insufficient immune reaction without affecting the antigen-specific immune response that is important for the patient's body in dealing with the infectious agent itself.

### Definitions:

An "immune response" relates to a response of the immune system whereby a change in number, change in rate of migration, or change in activation, of cells of the immune system occurs.

A "pathological" immune response relates to an immune response that is detrimental to the health of the individual and encompasses both over- and/or hyper-reactions of the immune system, which may in some cases lead to an inflammation, in addition to immune suppression or depression, in cases where the immune system does not react significantly when required.

An "acute lung disease" relates to an inflammatory disorder of the lung causing lung injury or lung failure, which occurs in an acute manner, for example developing within hours, days or not more than 1-2 weeks upon encounter with an adverse condition or injury. This condition might be an infection, trauma, burn, pankreatitis, uremia, transfusion related, disseminated coagulopathy, inhalation or aspiration of toxic or irritating substances, interstitial pneumonia related to different causes (the cause of the acute condition may include, but is not restricted to, Hamman-Rich Syndrome, acute eosinophilic pneumonia, acute episodes of either systemic lupus erythematodes, progressive systemic sclerosis, rheumatoid arthritis, dermatomyositis, Sjögren's syndrome, or respiratory bronchioloitis with interstitial lung disease [RB-ILD], desquamative interstitial pneumonia [DIP], lymphoid interstitial pneumonia [LIP]) or medical treatments such as mechanical ventilation that cause syndromes such as acute respiratory distress syndrome (ARDS), acute lung injury (ALI) due to local damage and inflammatory responses; it might include a combination thereof. In one embodiment a chronic condition may lead to the induction of (a distinct) acute condition. The acute condition is the indication to be addressed by the present invention. The acute lung diseases of the present invention differentiate themselves from chronic or allergic lung diseases such as asthma, airway hyperreactivity, COPD or chronic bronchitis by their pathologic and pathogenic nature, short induction times and non-repetitive frequency or recurrence in patients.

A "pathological immune response associated with acute lung disease" refers therefore to an immune response in a patient that is detrimental to the patients health, which has occurred together with, due to, simultaneously with, or after an acute lung disease.

In the case of infection, the condition to be treated directly in the present invention is however not the infectious agent itself, rather the associated immune response. In the case of IL-27 treatment (or with an IL-27 agonist) the immune response to be treated is an immunopathology, preferably an inflammatory immune response caused by the infectious lung disease, or in the case of the IL-27 antagonist, the immune suppression caused by an infectious lung disease, or conditions worsening immunopathology in the context of medical treatment, such as application of artificial ventilation.

In the case of non-infectious acute lung disease, the invention encompasses treatment of severe or even life-threatening immunopathology of the lung in acute lung disease, whereby the non-infectious lung disease leads to sterile inflammations of the lung, including those related to inhalative irritants, mechanical ventilation, trauma, burn, pankreatitis, uremia, transfusion related, disseminated coagulopathy, acid aspiration, idiopathic (Haman-Rich Syndrome) and any other sterile cause of ARDS (acute respiratory distress syndrome).

The term "influenza" relates to those viruses typically understood as influenza by one skilled in the art, for example RNA viruses that make up three of the five genera of the family Orthomyxoviridae: Influenzavirus A, Influenzavirus B, and Influenzavirus C. These viruses are related to the human parainfluenza viruses, which are RNA viruses belonging to the paramyxovirus family that are a common cause of respiratory infections in children such as croup, but can also cause a disease similar to influenza in adults. The type A influenza viruses are the most virulent human pathogens among the three influenza types and cause the most severe disease. The influenza A virus can be subdivided into different serotypes based on the antibody response to these viruses. The serotypes that have been confirmed in humans, ordered by the number of known human pandemic deaths, are: H1N1, which caused Spanish Flu in 1918, and Swine Flu in 2009, H2N2, which caused Asian Flu in 1957, H3N2, which caused Hong Kong Flu in 1968, H5N1, which caused Bird Flu in 2004, H7N7, which has unusual zoonotic potential, H1N2, endemic in humans, pigs and birds, H9N2, H7N2, H7N3, H10N7. Influenza B may also be treated according to the present invention, which almost exclusively infects humans and is less common than influenza A. Influenzavirus C can also be treated, which infects humans, dogs and pigs, sometimes causing both severe illness and local epidemics.

"Inflammatory immune response" means a disorder or pathological condition where the pathology results, in whole or in part, from, e.g., a change in number, change in rate of migration, or change in activation, of cells of the immune system. Cells of the immune system include, e.g., T cells, B cells, monocytes or macrophages, antigen presenting cells (APCs), dendritic cells, microglia, NK cells, NKT cells, innate lymphoid cells (ILC), neutrophils, eosinophils, mast cells, or any other cell specifically associated with the immune reaction, for example, cytokine-producing endothelial or epithelial cells. "Inflammatory immune response" may also mean a disorder or pathological condition where the pathology results, in whole or in part, from an increase in the number and/or increase in activation of cells of the immune system, e.g., of T cells, B cells, monocytes or macrophages, alveolar macrophages, dendritic cells, NK cells, NKT cells, ILC, neutrophils, eosinophils, or mast cells.

A "suppression of an immune response" relates to a endogenous suppression or depression of the normal immune response to infectious agents, which can be measured by a change in function, number, rate of migration, or activation of cells of the immune system.

"Inhibitors" and "antagonists" or "activators" and "agonists" refer to inhibitory or activating molecules, respectively, e.g., for the activation of, e.g., a ligand, receptor, cofactor, a gene, cell, tissue, or organ.

A "modulator" of, e.g., a protein, a gene, a receptor, a ligand, or a cell, is a molecule that alters an activity of the protein, gene, receptor, ligand, or cell, where activity can be activated, inhibited, or altered in its regulatory properties. The modulator may act alone, as an active agent itself, for example a protein or nucleic acid, or it may use a cofactor, e.g., a protein, metal ion, or small molecule.

Inhibitors are compounds that decrease, block, prevent, delay activation, inactivate, desensitize, or down regulate, e.g., a gene, protein, ligand, receptor, or cell.

Activators are compounds that increase, activate, facilitate, enhance activation, sensitize, or up regulate, e.g., a gene, protein, ligand, receptor, or cell. An inhibitor may also be defined as a composition that reduces, blocks, or inactivates a constitutive activity.

An "agonist" is a compound that is active itself or interacts with a target to cause or promote an increase in the activation of the target. A preferred embodiment of an IL-27 agonist is IL-27 protein itself, for example recombinant IL-27, or a nucleic acid that encodes L-27 which when expressed produces functional IL-27.

An "antagonist" is a compound that opposes the actions of an agonist. An antagonist prevents, reduces, inhibits, or neutralizes the activity of an agonist. An antagonist can also prevent, inhibit, or reduce constitutive activity of a target, e.g., a target receptor, even where there is no identified agonist.

"Administration" and "treatment," as it applies to an animal, human, experimental subject, cell, tissue, organ, or biological fluid, refers to contact of a pharmaceutical, therapeutic, diagnostic agent, compound, or composition to the animal, human, subject, cell, tissue, organ, or biological fluid. "Administration" and "treatment" can refer, e.g., to therapeutic, placebo, pharmacokinetic, diagnostic, research, and experimental methods. "Treatment of a cell" encompasses contact of a reagent to the cell, as well as contact of a reagent to a fluid, where the fluid is in contact with the cell. "Administration" and "treatment" also means in vitro and ex vivo treatments, e.g., of a cell, by a reagent, diagnostic, binding composition, or by another cell. "Treatment," as it applies to a human, veterinary, or research subject, refers to therapeutic treatment, prophylactic or preventative measures, to research and diagnostic applications.

"Treatment" as it applies to a human, veterinary, or research subject, or cell, tissue, or organ, encompasses contact of an IL-27 agonist or IL-27 antagonist to a human or animal subject, a cell, tissue, physiological compartment, or physiological fluid. "Treatment of a cell" also encompasses situations where the IL-27 agonist or IL-27 antagonist contacts IL-27 receptor (heterodimer of WSX-1/TCCR and gpBO), e.g., in the fluid phase or colloidal phase, as well as situations where the agonist or antagonist contacts a fluid, e.g., where the fluid is in contact with a cell or receptor, but where it has not been demonstrated that the agonist or antagonist contacts the cell or receptor. Preferably a therapeutically effective amount of IL-27 agonist or antagonist is applied according to the present invention.

"Effective amount" or "therapeutically effective amount" means an amount sufficient to ameliorate a symptom or sign of a disorder or physiological condition or an amount sufficient to permit or facilitate a diagnosis of the disorder or physiological condition. An effective amount for a particular patient or veterinary subject may vary depending on factors such as the condition being treated, the overall health of the patient, the method route and dose of administration and the severity of side effects. An effective amount can be the maximal dose or dosing protocol that avoids significant side effects or toxic effects. The effect will result in an improvement of a diagnostic measure, parameter, or detectable signal by at least 5%, usually by at least 10%, more usually at least 20%, most usually at least 30%, preferably at least 40%, more preferably at least 50%, most preferably at least 60%, ideally at least 70%, more ideally at least 80%, and most ideally at least 90%, where 100% is defined as the diagnostic parameter shown by a normal subject. "Effective amount" also relates to an amount of an IL-27 agonist, antagonist, or binding compound or composition, sufficient to allow or facilitate the diagnosis of a symptom or sign of a disorder, condition, or pathological state.

"Expression" refers to a measure of mRNA or polypeptide encoded by a specific gene. Units of expression may be a measure of, e.g., the number of molecules of mRNA or polypeptide/mg protein, the number of molecules of mRNA or polypeptide/cell, in measurements of expression by cell, tissue, cell extract, or tissue extract.

### Abbreviations

- BAL: Bronchoalveolar lavage
- IL-10R: Interleukin 10 receptor
- PFU: Plaque forming units
- dLN: Lung draining lymph nodes
- EB13: Epstein-Barr inducible gene 3
- ICS: Intracellular staining
- p.i.: post infection

### FIGURES

The invention is further described by the figures. These are not intended to limit the scope of the invention.

### Brief description of the Figures:

Figure 1. Absence of IL-27Ra leads to increased mortality and immunopathology during influenza
Figure 2. Absence of IL-27Ra leads to increased numbers of IFN-y and IL-17-producing CD4+ and CD8+ T cells in the respiratory tract
Figure 3. Induction of IL-10 in CD4+ T cells is dependent on IL-27Ra signalling
Figure 4. IL-27 regulates IL-17 response via IL-10, while IFN-y production by CD4+ and CD8+ T cells is directly modulated by IL-27 signalling
Figure 5. rIL-27 treatment at an early stage of influenza virus infection aggravates disease severity and impairs viral clearance
Figure 6. Delayed rIL-27 treatment alleviates immunopathology
Figure 7. Increased TNF-α response in influenza virus-infected Il27ra-/- mice
Figure 8. IL-10 production by CD4+ T cells in mice infected with influenza virus is dependent on IL-27Rα signalling
Figure 9. Blocked IL-10 signaling during influenza impaired IL-17 regulation in CD4+ and CD8 T cells
Figure 10. Direct IL-27Rα signaling and not IL-10 regulates IFN-y production by CD8+ T cells during influenza virus infection
Figure 11. rIL-27 treatment during the later phase of influenza does not affect antigen-specific CD4+ T cell cytokine responses

Detailed description of the figures:

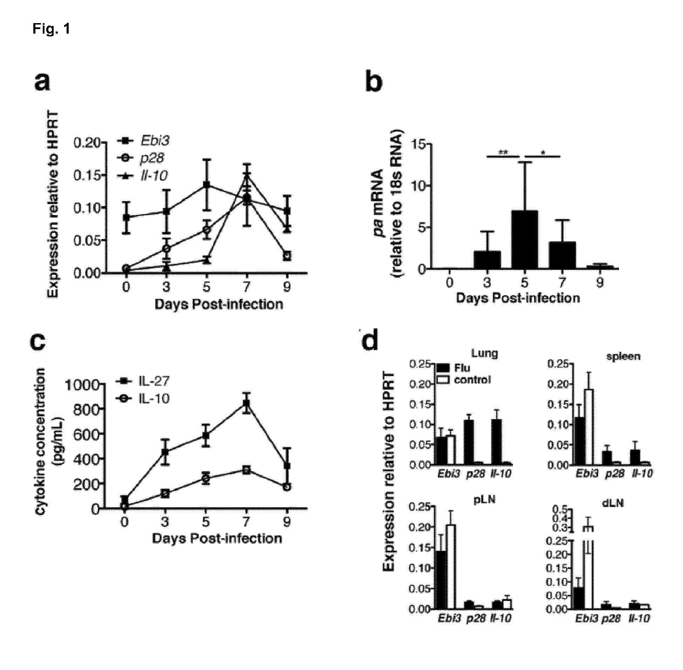

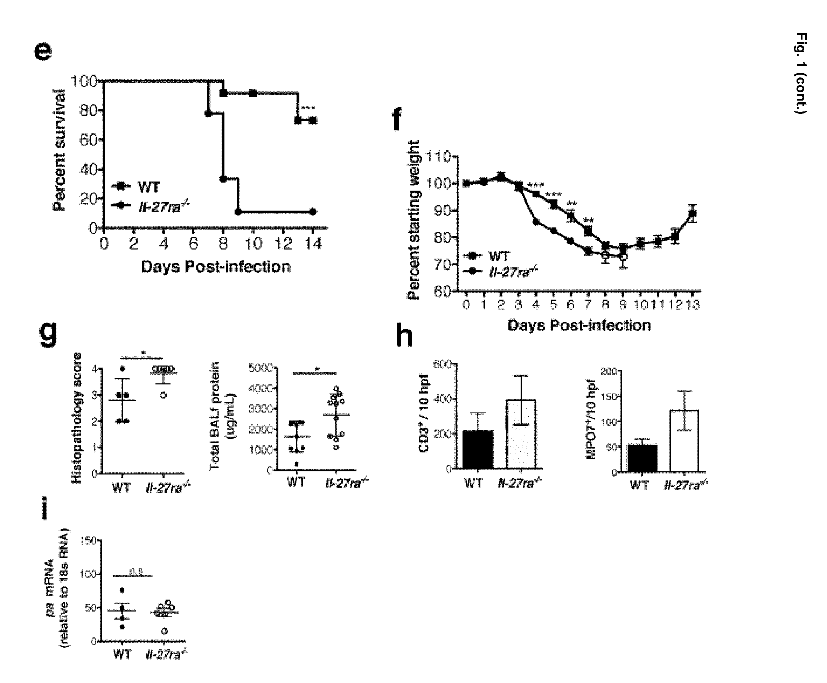

Figure 1. Absence of IL-27Ra leads to increased mortality and immunopathology during influenza. (A) Il-27p28, Ebi3, Il-10 mRNA and (B) influenza virus polymerase (pa) mRNA in the lungs of infected C57BL/6 mice were analyzed by qRT-PCR at indicated days p.i. (C) cytokine concentration in the lungs were quantified by ELISA (D) Il-27p28, Ebi3 and Il-10 mRNA in lungs, spleen, peripheral (pLN) and lung-draining (dLN) lymph nodes of infected and uninfected C57BL/6 mice were analyzed by qRT-PCR at day 7 p.i. (E) Survival and (F) weight loss of infected Il-27rai- (n = 9) and wild-type (WT) C57BL/6 (n = 12) mice after a low dose influenza virus challenge. Open circles represent remaining Il-27rai- mice (n = 2) (G) Pathological scores of H&E-stained lungs of II-27rai- and WT mice at day 7 p.i. (left). Protein content in the BAL fluid of Il-27ra-/- and WT mice at day 9 p.i. was quantified by BCA (right). (H) IHC staining for lung-infiltrating CD3+ cells and neutrophils (MPO7+) in Il-27ra-/- (n = 6) and WT (n = 5) mice at day 7 p.i. (I) Viral pa mRNA in lungs of Il-27ra-/- mice were analyzed by qRT-PCR at day 7 p.i. All data sets were pooled from at least two independent experiments with similar results. Values represent means ± SD. P values for E were determined by log-rank survival test. P values for B, F, G and I were determined by unpaired two-tailed Student's t test. *P<0.05; **P<0.01; ***P<0.0001; ns, not significant.

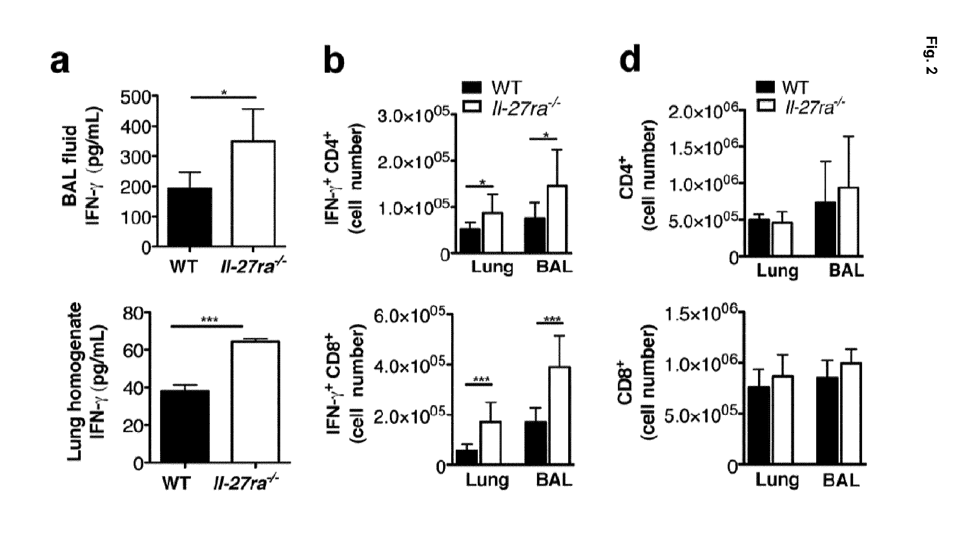

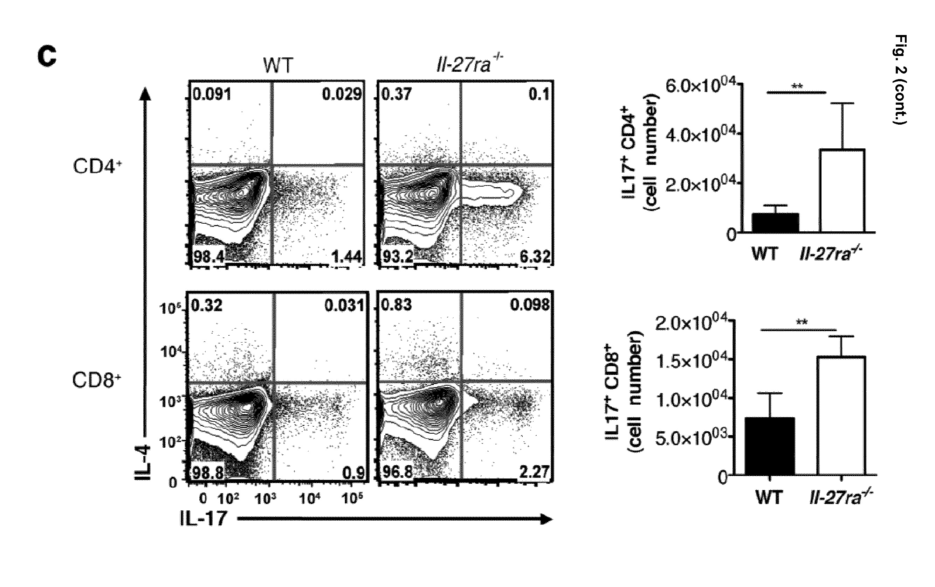

Figure 2. Absence of IL-27Ra leads to increased numbers of IFN-y and IL-17-producing CD4+ and CD8+ T cells in the respiratory tract. Il-27rai- and wild-type (WT) C57BL/6 mice were infected with a low dose influenza virus. At day 9 p.i., (A) ELISA was performed to measure IFN-y concentrations in the BAL fluid and lung homogenates of Il-27rai- (n = 6) and WT (n = 6) mice. Intracellular staining (ICS) of lung and BAL cells of Il-27ra-/- (n = 8) and WT (n = 8) mice were performed after in vitro restimulation with PMA/ionomycin to measure the numbers of (B) IFN-y and (C) IL-17 and IL-4-producing CD4+ or CD8+ T cells by FACS. (D) Total numbers of CD4+ and CD8+ T cells in the lungs and BAL of infected Il-27ra-/- and WT mice were quantified by FACS. All data sets were pooled from at least two independent experiments with similar results. P values were determined by unpaired twotailed Student's t test. Values are means ± SD. *P<0.05; **P<0.01; ***P<0.0001.

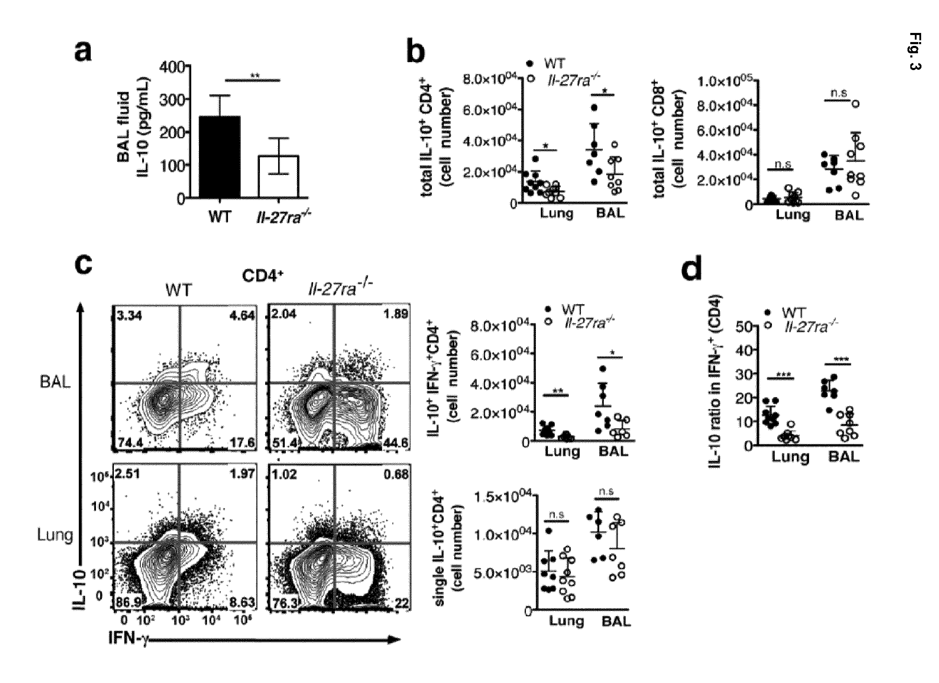

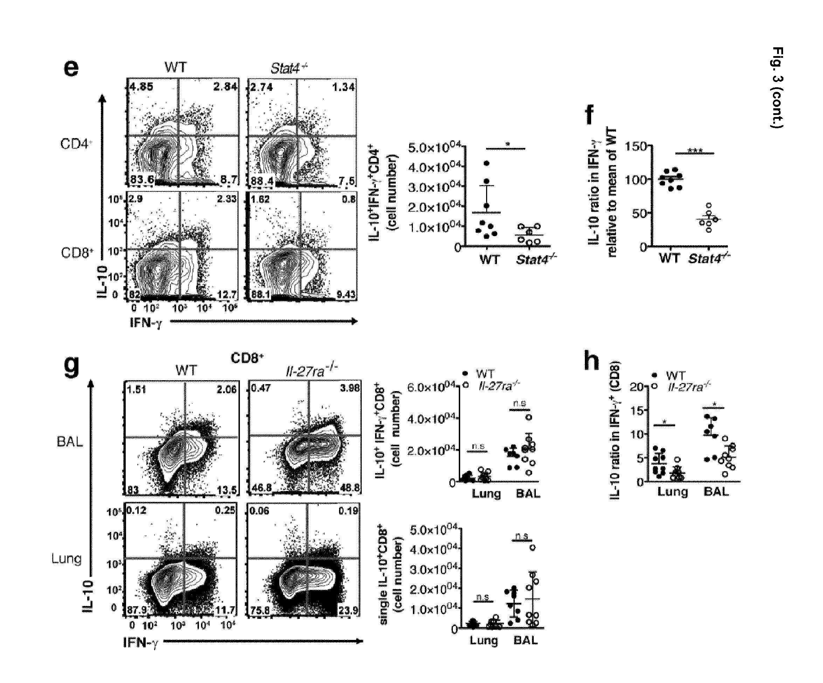

Figure 3. Induction of IL-10 in CD4+ T cells is dependent on IL-27Ra signalling. Il-27ra-/- and wild-type (WT) C57BL/6 mice were infected with a low dose influenza virus. At day 9 p.i, (A) levels of IL-10 in the BAL fluid were determined by ELISA and (B) numbers of total IL-10-producing T cells or IL-10+IFN-y+ double-producing (C) CD4+ or (G) CD8+ T cells were analyzed by FACS after restimulation with PMA/ionomycin in vitro. The ratios of IL-10:IFN-y in gated (D) CD4+ or (H) CD8+ T cells in the lungs and BAL are indicated. (E) Stat4-/- and wild-type (WT) BALB/c mice were infected with a low dose influenza virus, then ICS was performed to determine IL-10 and IFN-y producing-CD4+ or CD8+ T cells in the lungs after restimulation with PMA/ionomycin. (F) IL-10:IFN-y ratios in gated CD4+ T cells of influenza-infected Stat4-/- or WT mice are shown. All data sets were pooled from at least two independent experiments with similar results. P values were determined by unpaired twotailed Student's t test. Values are means ± SD. *P<0.05; **P<0.01; ***P<0.0001; ns, not significant.

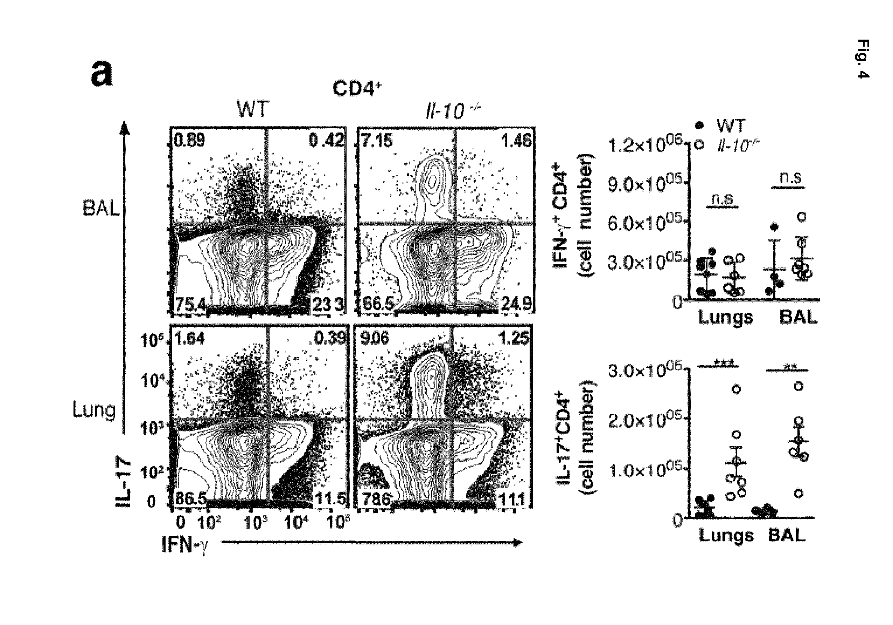

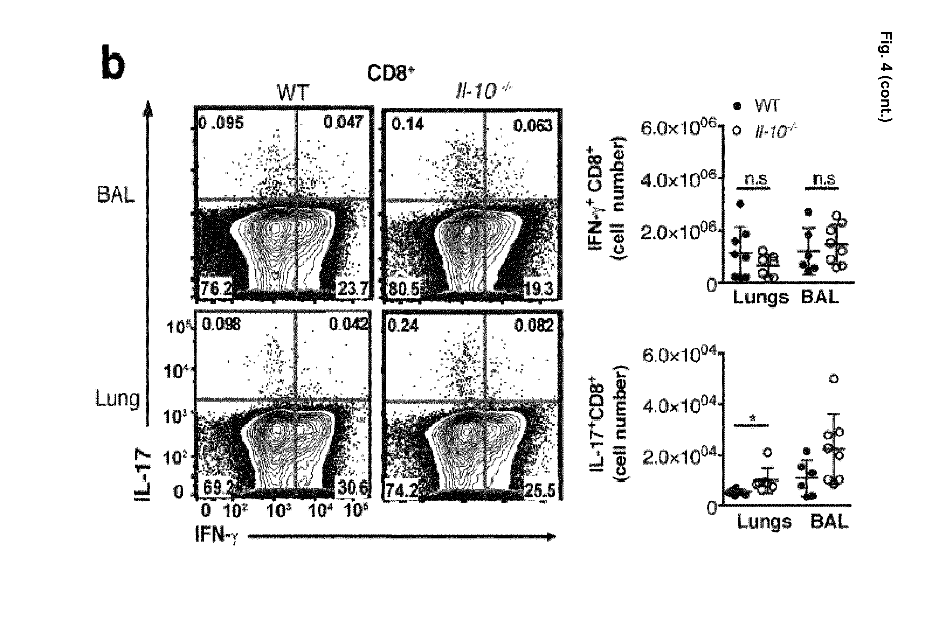

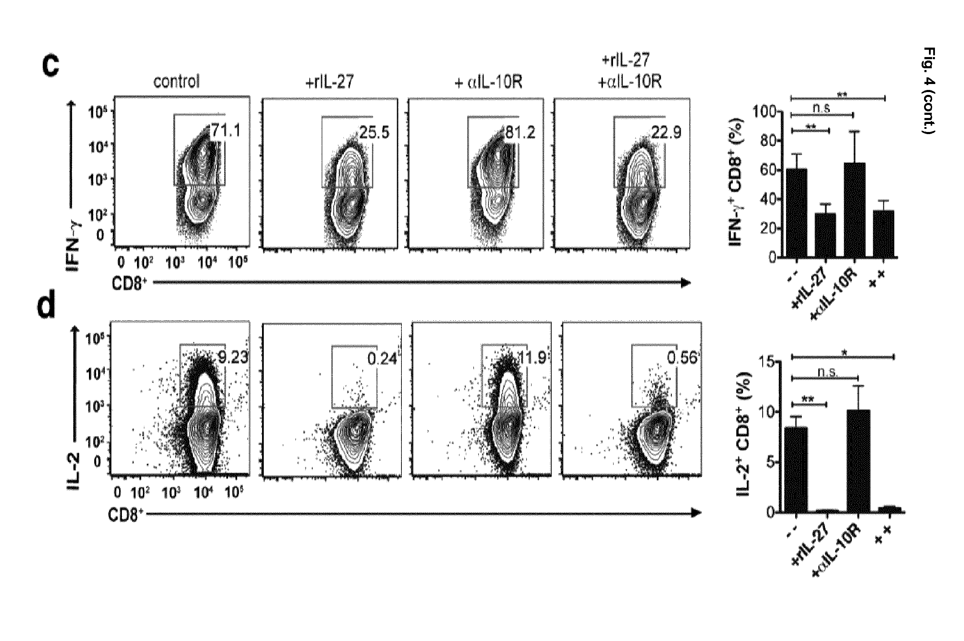

Figure 4. IL-27 regulates IL-17 response via IL-10, while IFN-y production by CD4+ and CD8+ T cells is directly modulated by IL-27 signalling. Il-10-/- and wild-type (WT) C57BL/6 mice were infected with a low dose influenza virus. At day 7 p.i., numbers of antigen-specific IFN-y and IL-17-producing (A) CD4+ or (B) CD8+ T cells in the lungs and BAL were assessed by FACS after in vitro restimulation with influenza peptides. (C and D) Naive CD8+ T cells were activated with plate-bound anti-CD3 and anti-CD28 in Tc1 polarizing conditions. Cells were incubated in the presence or absence of rIL-27 and/or anti-IL-10 receptor blocking antibody (aIL-10R) for 3 days then transferred to plates without anti-CD3 and anti-CD28. Media were replenished with IL-2, rIL-27 and/or ctIL-10R antibody for additional 2 days, for a total of 5 days in culture. Cells were restimulated with PMA/ionomycin and analyzed by FACS after ICS. A and B were pooled from two independent experiments with similar results. C and D represent data from three independent experiments with similar results. P values were determined by unpaired two-tailed Student's t test. Values are means ± SD. Where *P<0.05; **P<0.01; ***P<0.0001; ns, not significant.

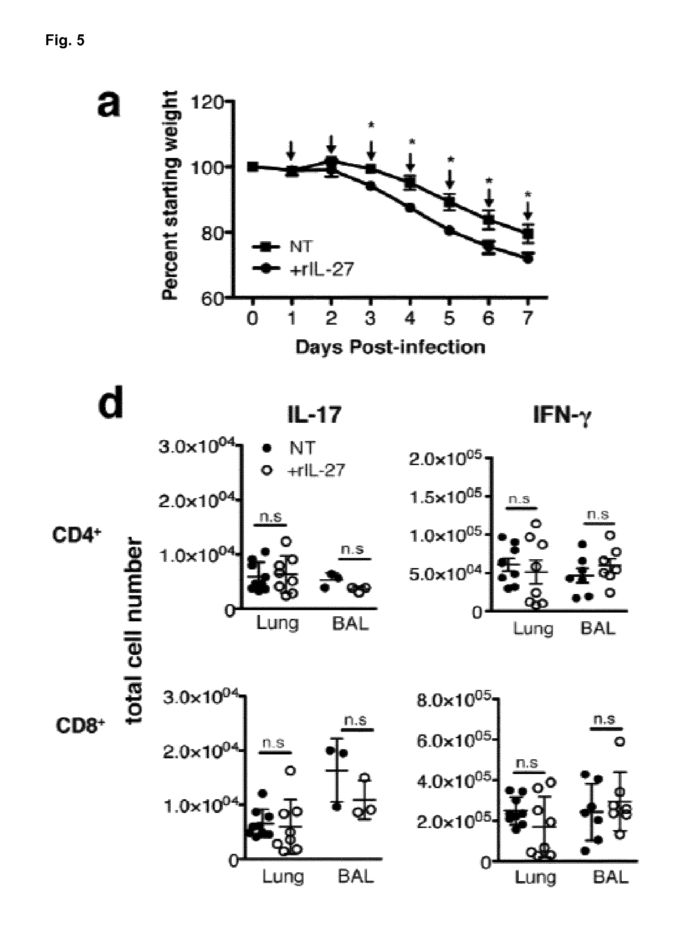

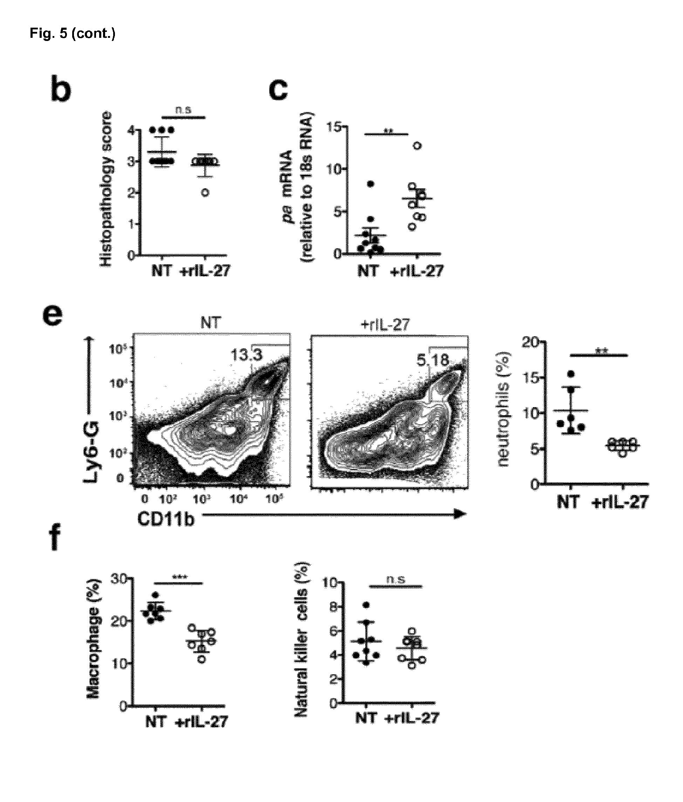

Figure 5. rIL-27 treatment at an early stage of influenza virus infection aggravates disease severity and impairs viral clearance. C57BL/6 mice were challenged with an intermediate dose of influenza virus then treated daily with rIL-27 from day 1 to day 7 p.i. (A) Weight loss of rIL-27 treated (n = 5) and non-treated (NT) (n = 5) mice are plotted. Arrows (.L) indicate point of treatment. At day 7 p.i., (B) histological scores of H&E-stained lungs of treated and NT mice were determined, (C) viral pa mRNA expression was determined by qRT-PCR, (D) numbers of antigen-specific IL-17 and IFN-y-producing T cells, (E) neutrophils and (F) macrophage and NK cells in the lungs were determined by FACS. Gated cells in FACS plots indicate the percentage of neutrophils in total lung cells. Data from A represents data from at least two independent treatment experiments, data from B, C, D, E and F are pooled from at least two independent experiments yielding similar results. P values were determined by unpaired two-tailed Student's t test. Values are means ± SD. *P<0.05; **P<0.01; ***P<0.0001; ns, not significant.

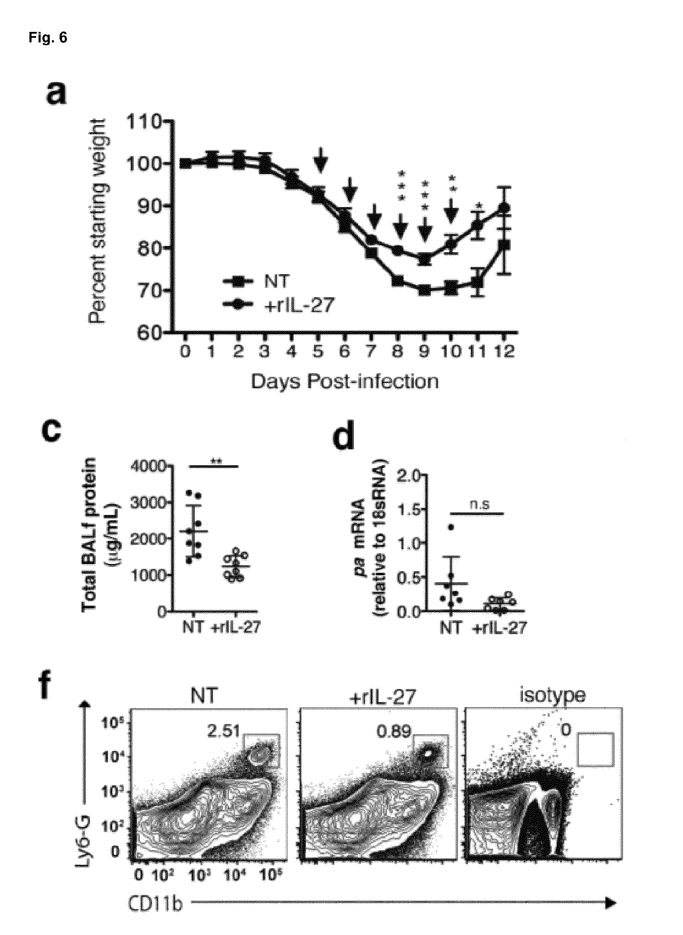

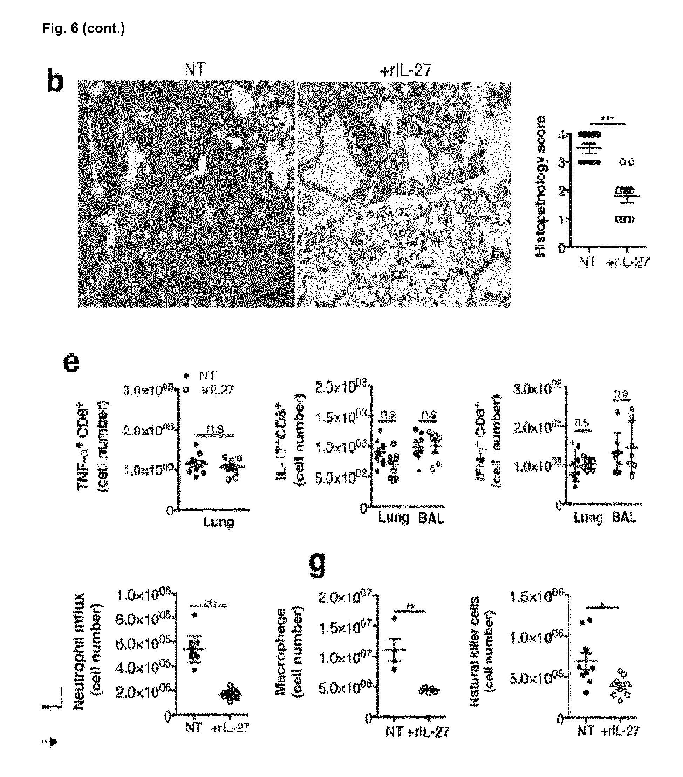

Figure 6. Delayed rIL-27 treatment alleviates immunopathology. C57BL/6 mice were challenged with an intermediate dose of influenza virus then treated daily with rIL-27 from day 5 to day 10 p.i. (A) Weight loss of rIL-27 treated non-treated (NT) mice is plotted. Arrows (.L) indicate points of treatment. At day 9 p.i., (B) histological comparison of H&E-stained lungs was performed, (C) protein content in the BAL fluid was measured by BCA, (D) viral polymerase (pa) mRNA expression in the lungs was measured by qRT-PCR, (E) antigen-specific cytokine production by CD8+ T cells, (F) neutrophils (G) macrophages and NK cells in lungs were determined by FACS. Values for the weight loss curves are data pooled from at least two independent experiments, representing the means ± SD of the following numbers of mice per group: day 1-9, n = 14; day 10-11, n = 7; day 12, n = 6. Data from B to G are pooled from at least two independent experiments with similar results. P values were determined by unpaired two-tailed Student's t test. Values are means ± SD. *P<0.05; **P<0.01; ***P<0.0001; ns, not significant.

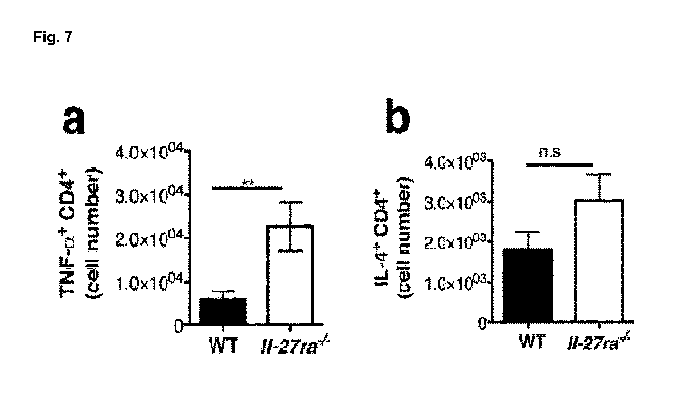

Figure 7. Increased TNF-α response in influenza virus-infected Il27ra-/- mice. Intracellular staining (ICS) of Il-27ra-/- (n = 7) or C57BL/6 (n = 6) lung cells after in vitro restimulation with PMA/ionomycin to determine accumulation of (A) TNF-α and (B) IL-4-producing CD4+ T cells at day 9 p.i. P values were determined by unpaired two-tailed Student's t test. Values are means ± SD. *P<Ø.Ø5; **P<Ø.Ø1; n.s, not significant.

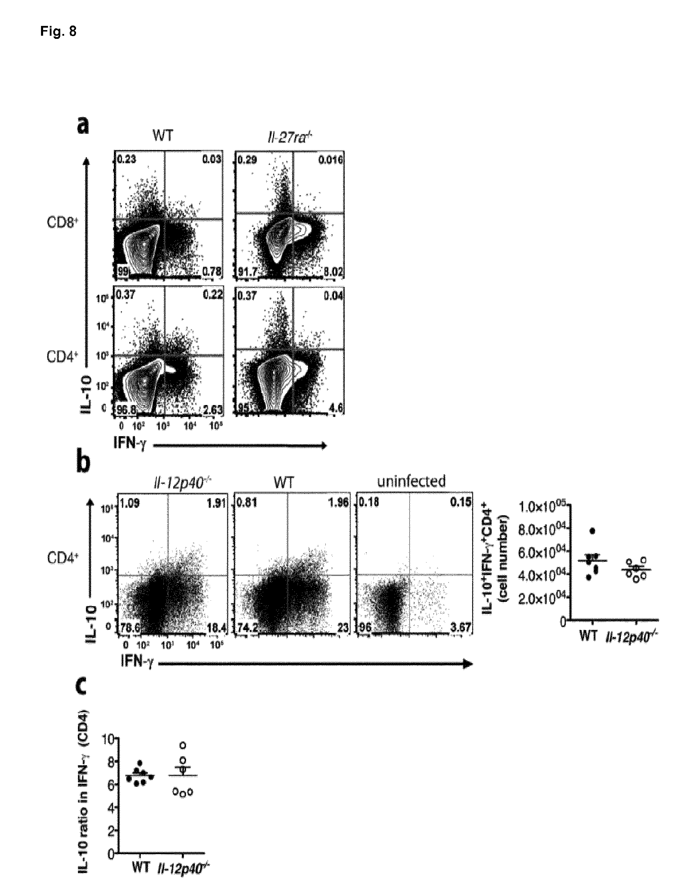

Figure 8. IL-10 production by CD4+ T cells in mice infected with influenza virus is dependent on IL-27Rα signalling. Il-27ra-/-, Il-12p40-/- and wild-type (WT) mice were infected with a low dose influenza virus. At day 9 p.i., IL-10 and IFN-y-producing CD4+ or CD8+ T cells in the (A) dLN of Il-27ra-/- or (B) lungs of Il-27p40-/- were analyzed by FACS after in vitro restimulation with PMA/ionomycin. (C) Ratios of IL-10:IFN-y in gated CD4+ T cells from lungs of infected Il-12p40-/- at day 9 p.i. are shown. All data sets represent data from two independent experiments with similar results. Data shown in B and C are pooled values from two independent experiments. P values were determined by unpaired two-tailed Student's t test. Values are means ± SD. *P<0.05; **P<0.01; n.s, not significant.

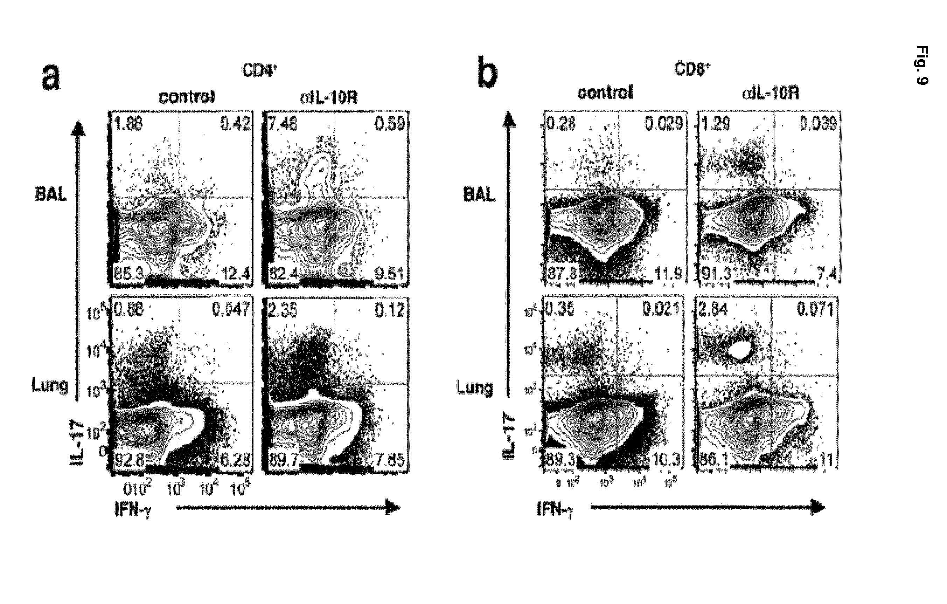

Figure 9. Blocked IL-10 signaling during influenza impaired IL-17 regulation in CD4+ and CD8 T cells. (A and B) C57BL/6 mice were infected with a low dose influenza virus then administered with anti-IL-10 receptor blockingantibody (αIL-10R). Antigen-specific cytokine production by CD4+ or CD8+ T cells was detected through FACS after in vitro restimulation with influenza virus peptides. All data sets represent data obtained from at least two independent experiments with similar results.

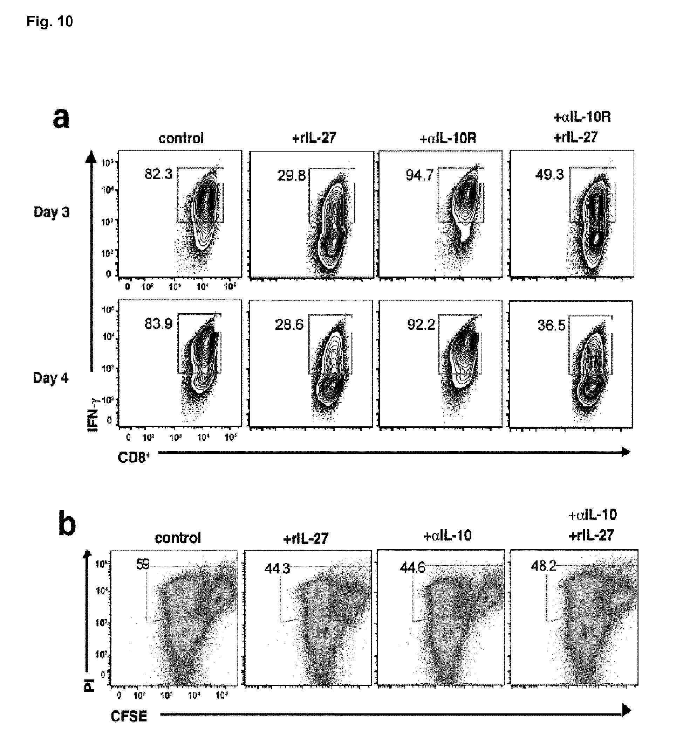

Figure 10. Direct IL-27Rα signaling and not IL-10 regulates IFN-y production by CD8+ T cells during influenza virus infection. (A) Naive CD8+ T cells were activated by plate-bound anti-CD3 and anti-CD28 in Tc1 polarizing conditions and were supplemented with rIL-27 and/or αIL-10R antibody. After 3 days in culture, cells were re-transferred to plates without stimuli then media was added with rIL-2 and IL-27 and/or αIL-10R antibody for an additional 2 days for a total of 5 days in culture. ICS was performed to detect IFN-y-producing Tc1 cells in various conditions. (B) Previously CFSE-labelled Tc1 cells were cultured as mentioned above then stained with propidium iodide (PI) prior to FACS acquisition. All data sets represent data obtained from at least two independent experiments with similar results.

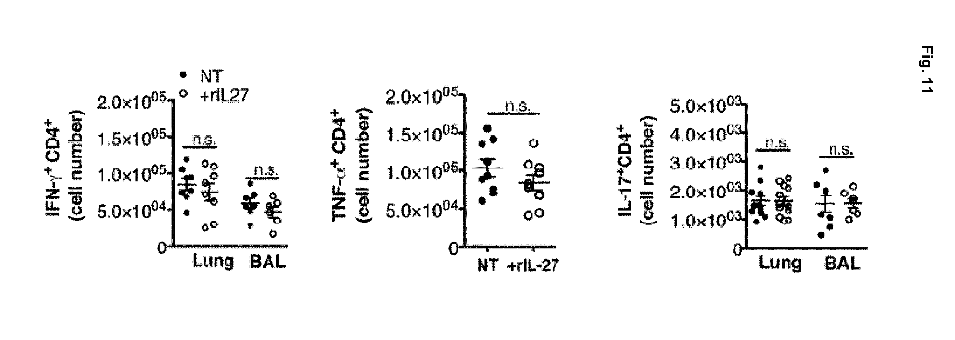

Figure 11. rIL-27 treatment during the later phase of influenza does not affect antigen-specific CD4+ T cell cytokine responses. C57BL/6 mice were infected with an intermediate dose of influenza virus then administered with rIL27 daily from day 5 to day 9. At day 9 p.i., antigen-specific cytokine production by CD4+ T cells were detected through FACS after restimulation with influenza virus peptides. Data shown are pooled from at least two independent experiments with similar results. P values were determined by unpaired two-tailed Student's t test. Values are means ± SD. *P<0.05; **P<0.01; n.s, not significant.

### EXAMPLES

The invention is further described by the following examples. These are not intended to limit the scope of the invention. The methods described herein were used in carrying out the present invention as demonstrated in the examples. They are intended to further describe the invention by way of practical example and do not represent a limiting description of the invention.

### Impaired IL-27Ra signaling leads to increased mortality following influenza virus challenge

To determine the role of IL-27 in shaping the immune response against influenza virus, we first examined the kinetics of Il-27p28 and Ebi3 mRNA expression in the lungs following low-dose influenza virus infection (Fig. 1A). While Ebi3 was not significantly upregulated in the lungs, Il-27p28 displayed a pronounced peak at day 7, two days after the peak of the viral load (Fig. 1B). Coinciding with the peak of Il-27p28 expression was the maximal expression of Il-10 mRNA, which is consistent with the assumption that IL-27 is an important inducer of IL-10 (Anderson et al., 2009; Awasthi et al., 2007; Fitzgerald et al., 2007; Stumhofer et al., 2006). Quantifying the cytokines at the protein level, we observed a strong up-regulation of IL-27 and IL-10 in the lungs at day 7 p.i, confirming the mRNA data (Fig. 1C). Analyzing the lungs, spleen, peripheral (pLN) and lung-draining (dLN) lymph nodes, we found that while Ebi3 is constitutively expressed in most organs, the strong upregulation of Il-27p28 and Il-10 mRNA was seen predominantly in the lungs and to a lesser extent in the spleen (Fig. 1D).

To assess the impact of IL-27 on survival during influenza, we challenged wild-type (WT) C57BL/6 or IL-27 receptor-deficient (Il-27ra-/-) mice with a low virus dose. Following infection, we found that abrogated IL-27Rα signaling resulted in a dramatic increase in mortality compared to WT mice (Fig. 1 E). This increase correlated with a greater weightloss among the knockout mice relative to WT during the course of infection (Fig. 1 F). We next examined whether the increased mortality of Il-27ra-/- mice was related to differential pathology. Histological findings revealed interstitial thickening of the alveolar walls, edema, perivascular infiltration and necrotizing bronchitis by day 7 p.i. in both mouse strains (data not shown); however Il-27ra-/- mice developed a more severe lung pathology compared to control mice at day 9 p.i (Fig. 1 G). Measurement of the total protein in the bronchoalveolarlavage (BAL) fluid suggested increased capillary leakage in the respiratory tract of infected Il27ra-/- mice compared to controls, supporting the histological findings (Fig. 1G). Simultaneously, higher infiltration of CD3+ cells and neutrophils (MPO7+) were found in the lungs of Il-27ra-/- mice (Fig. 1H). The increase in immunopathology and mortality was not due to a compromised elimination of virus in Il-27ra-/- mice, as virus load was not significantly different between Il-27ra-/- and control mice (Fig. 1I). These findings suggest that IL-27 plays a critical role in limiting immunopathology during the later stages of infection.

### Compromised regulation of IFN-γ and IL-17 production in influenza virus-infected Il-27ra-/- mice

As IL-27 is able to suppress both TH1 and TH17 responses in models of autoimmunity or infection (Batten et al., 2006; Fitzgerald et al., 2007; Holscher et al., 2005; Stumhofer et al., 2006), we asked whether the absence of IL-27 receptor would result in dysregulated inflammatory cytokine production during the later stages of influenza, where viral loads are almost cleared. We infected Il-27ra-/- mice with a low virus dose and analyzed the numbers of infiltrating IFN-γ, IL-17, TNF-α and IL-4-producing CD4+ and CD8+ T cells at day 9 p.i. Correlating with the significant increase of IFN-γ levels in the BAL fluid and in lung homogenates of Il-27ra-/- mice (Fig. 2A), intracellular cytokine staining (ICS) revealed a dramatic increase in the numbers of IFN-γ-producing CD4+ and CD8+ T cells in these sites (Fig. 2B). The results suggest that IL-27Rα signaling regulates the accumulation of IFN-γ- producing T cells after primary influenza virus challenge.

Consistent with the ability of IL-27 to inhibit IL-17 responses in T cells (Batten et al., 2006; Stumhofer et al., 2006), we observed increased numbers of IL-17+CD4+ T cells in the lungs of Il-27ra-/- mice (Fig. 2C). Interestingly, IL-17 production by CD8+ T cells was also augmented when IL-27Rα was absent (Fig. 2C), suggesting that the regulatory function of IL-27/IL27Rα signaling extends to IL-17 production in CD8+ T cells. Additionally, we observed substantially higher numbers of TNF-α+CD4+ T cells in the lungs of mice lacking the IL27Rα receptor (Fig. 7A). In contrast to the observed increase of TH1-related cytokines, we detected no significant change in numbers of IL-4+CD4+ T cells infiltrating the lungs of infected Il-27ra-/- mice, indicating that the adaptive response remains polarized to TH1 (Fig. 2C and Fig. 7B). Moreover, we found comparable numbers of CD4+ and CD8+ T cells in the lungs of both strains at day 9 p.i. (Fig. 2D). Collectively, these data suggest a regulatory function for IL-27Rα in limiting IFN-γ and IL-17 production by both CD4+ and CD8+ T cells during influenza. γ

### CD4+ T cell production of IL-10 is regulated by the IL-27Rα receptor and partially via STAT4 signaling

Several studies have emphasized the role of IL-27 in promoting IL-10 secretion by IFN-γ+CD4+ T cells in vitro and in in vivo models of parasitic infection (Anderson et al., 2009; Awasthi et al., 2007; Stumhofer et al., 2006). Therefore, we sought to examine its role in inducing IL-10 in CD4+ T cells after influenza virus challenge. We infected Il-27ra-/- and WT mice with a low virus dose and analyzed the numbers of IL-10 and IFN-γ producing-CD4+ and CD8+ T cells in the lungs and BAL. Correlating with the decreased levels of IL-10 in the BAL fluid of infected Il-27ra-/- mice (Fig. 3A), we observed a substantial decrease in numbers of total IL-10+CD4+ T cells, but not of IL-10+CD8+ T cells, in the lungs and BAL of II-27ra-/- mice after in vitro restimulation with PMA/ionomycin (Fig. 3B). Notably, II-27ra-/- mice had reduced numbers of IL-10+IFN-γ+ double-positive CD4+ T cells in the lungs, BAL and dLN (Fig. 3C and Fig. 8A), suggesting that induction of these double-producing cells is dependent on IL-27Rα signaling. Furthermore, we observed that the strong increase in numbers of IFN-γ+CD4+ T cells in Il-27ra-/- mice was not accompanied by an increase in numbers of IL-10+IFN-y+CD4+ T cells. This observation is reflected in a significant reduction in the IL-10:IFN-y ratio in CD4+ T cells of Il-27ra-/- mice, in contrast to WT animals (Fig. 3D). It has to be noted, however, that IL-10iFN-y+ double-positive or IL-10 single-positive CD4+ cells were not completely lacking in Il-27ra-/- mice, suggesting that other factors besides IL-27 contribute to the induction of IL-10.

Previous in vitro studies demonstrated the importance of STAT4 signaling in promoting IL-10 in CD4+ T cells (Saraiva et al., 2009). To test whether STAT4 signaling is necessary in promoting IL-10 production by CD4+ T cells during influenza, we infected Stat4-/- mice with a low dose of influenza virus. We observed significantly fewer lung-infiltrating IL-10+IFN-y+ double-positive CD4+ and CD8+ T cells in infected Stat4-/- mice (Fig. 3E). Moreover, the IL10:IFN-y ratio of CD4+ T cells was substantially decreased in comparison to WT animals (Fig. 3F). As with IL-27 signaling, the absence of STAT4 did not completely abrogate the presence of IL-10+IFN-y+CD4+ T cells, suggesting the presence of other IL-10-inducing pathways. IL-12, a major cytokine signaling through STAT4 has been shown to support IL-10 generation in CD4+ T cells in other settings (Dong et al., 2007; Saraiva et al., 2009). However, CD4+ T cells from influenza virus-infected Il-12p40-/- mice have WT levels of IL-10 and a similar IL-10:IFN-y ratio relative to WT mice (Fig. 8B and C). These results suggest that induction of IL-10 in IFN-y+CD4+ T cells is partially mediated by STAT4 signaling but is independent of IL-12.

In contrast to the observed decrease in IL-10 production by CD4+ T cells, the numbers of IL10+IFN-y+ double-positive and IL-10 single-positive CD8+ T cells recovered from the lungs and BAL of influenza virus-infected II-27ra-/- mice were similar to WT animals (Fig 3G). Similar to CD4+ T cells, we observed a dramatic increase of IFN-y production by CD8+ T cells in the infected respiratory tract of II-27ra-/- mice, resulting in decreased IL-10:IFN-y ratios (Fig 3H). Taken together, these data demonstrate that induction of IL-10 in IFNγ+CD4+ T cells during influenza is driven by IL-27 and partially involves STAT4 signaling, but it is independent of IL-12. In contrast to CD4+ T cells, signaling through IL-27Rα is dispensable in promoting IL-10 production in CD8+ T cells.

### IL-10 regulates IL-17, while IL-27 signaling directly modulates IFN-γ production by CD4+ and CD8+ T cells

Next, we asked whether the increased IFN-γ production by CD4+ and CD8+ T cells in infected Il-27ra-/- mice is due to the lack of direct IL-27Rα signaling or absence of IL-27-induced IL-10. To address this question, we infected Il-10-/- mice with a low dose of influenza virus and analyzed the lungs and BAL for infiltrating IFN-γ and IL-17-producing T cells. In contrast to Il-27ra-/- mice, infected Il-10-/- mice displayed no change in numbers of IFN-γ-producing T cells in the lungs and BAL on day 7 p.i. (Fig. 4A and B). However, we found elevated numbers of IL-17+CD4+ T cells in the lungs and BAL, as well as slightly increased IL17+CD8+ T cells in the lungs of infected Il-10-/- mice (Fig. 4A and B). These data suggest that IL-10 limits IL-17 production but does not regulate the IFN-γ response of CD4+ and CD8+ T cells during influenza virus infection. These findings were verified by blocking IL-10 signaling in vivo by administration of an anti-IL-10 receptor monoclonal antibody (αIL-10R) to mice undergoing low-dose influenza virus infection. Similar to Il-10-/- mice, we observed an increase in frequencies of IL-17+CD4+ and IL-17+CD8+ T cells in the lungs and BAL of αIL-10R-treated mice at day 7 p.i. while IFN-γ production in the lung and BAL-infiltrating CD4+ and CD8+ T cells were not altered (Fig 9A and B). These results suggest that IL-27-induced IL-10 modulates IL-17 production, while IFN-γ secretion in T cells during influenza is directly regulated by IL-27.

To confirm a direct regulation of IFN-γ production in CD8+ T cells by IL-27, we examined its effect in vitro. Addition of recombinant IL-27 (rIL-27) significantly suppressed IFN-γ production by activated IFN-γ+CD8+ T cells (Tc1 cells) (Fig. 4C). Since it has been described that IL-27 induces IL-10 in CD8+ T cells in in vitro settings (Takeda et al., 2003), we blocked IL-10 signaling by addition of an aIL-10R monoclonal antibody. This treatment only slightly enhanced IFN-γ production, suggesting that IL-27 directly suppresses IFN-γ production in Tc1 cells in vitro. This was consistently found at multiple time-points examined over a 5-day period (Fig. 10A). Furthermore, we observed that addition of rIL-27 also suppressed endogenous IL-2 production in Tc1 cells (Fig. 4D). The observed decrease in IFNγ was not due to suppressed IL-2 production or activation-induced cell death (AICD), as exogenous IL-2 was added to the cultures, and propidium iodide staining did not indicate increased apoptosis due to the addition of rIL-27 (Fig. 10B). Taken together, these data demonstrate that the modulation of IFN-y responses in CD4+ and CD8+ T cells is a direct effect of IL-27/IL-27Rα signaling and not mediated through IL-10.

### Treatment with rIL-27 alleviates immunopathology when administered at later time points during influenza

Having demonstrated a pronounced effect of IL-27Rα signalling in regulating cytokine production and immunopathology, we wondered whether this property could be exploited for therapeutic purposes. To test whether early treatment with rIL-27 is advantageous for mice infected with an intermediate virus dose, we administered the cytokine intraperitoneally (i.p.), 24 hours after infection and then daily until day 7 p.i. IL-27-treated mice exhibited marked increase in weight loss (Fig. 5A) and reached the cutoffs for conditional survival by day 7. Histopathological findings revealed no significant difference in the pathological score between treated and non-treated mice (Fig. 5B). We next examined viral burden and found that early rIL-27-treated mice had significantly increased viral loads in comparison to non-treated (NT) mice (Fig. 5C). To determine whether impaired viral clearance is due to suppressed cytokine response by T cells, we analyzed T cells after restimulation with a mixture of immunodominant influenza peptides. No difference in the numbers of infiltrating antigen-specific IL-17 and IFN-y-producing CD4+ and CD8+ T cells were observed (Fig. 5D). As neutrophils and macrophages play an essential role in viral clearance during influenza (La Gruta et al., 2007), we analyzed the frequencies of these cells in the lungs. We found that mice treated with rIL-27 exhibited a drastic reduction of infiltrating neutrophils (Fig. 5E) and monocytes, but not of NK cells (Fig. 5F), suggesting that rIL-27 treatment during the early stages of influenza suppresses neutrophil and macrophage influx which can negatively impact viral clearance and lead increased mortality.

As endogenous IL-27 peaks in the later phase of influenza, it is conceivable that its physiological absence in the early phase of viral infection allows for a vigorous defense reaction, but delayed production serves to suppress exaggerated immunopathology in the later phase of infection. Thus, we administered rIL-27 daily from day 5 to day 10 p.i. In these experiments, treated mice displayed significantly decreased weight loss compared to the nontreated groups (Fig. 6A). Consistent with this, histopathological analysis revealed significantly reduced immunopathology (Fig. 6B) and decreased protein content in the BAL fluid upon rIL-27 treatment, indicating reduced capillary leakage in the infected respiratory tract (Fig. 6C). Moreover, rIL-27-treated mice appeared to have similar, if not better, control of viral loads when compared to the non-treated group (Fig. 6D), suggesting that viral clearance is not compromised by the treatment. We observed no differences in the numbers of antigen-specific TNF-a, IL-17 and IFN-y-producing CD8+ T cells from the lung and BAL of treated and non-treated mice (Fig. 6E). Similarly, the numbers of infiltrating TNF-a, IL-17 and IFN-y-producing CD4+ T cells were comparable between both groups (Fig. 11 indicating that delayed treatment with rIL-27 minimally affects cytokine production by CD4+ and CD8+ T cells. In contrast to the unchanged adaptive immune response, systemic administration of rIL-27 resulted in significantly decreased lung-infiltrating neutrophils (Fig. 6F), macrophages and NK cells (Fig. 6G). These data correlate with the ability of IL-27 to act as a negative regulator of innate effector cells such as neutrophils and macrophages (Hamano et al., 2003; Li et al., 2010; Passos et al., 2010; Wirtz et al., 2006; Yoshida and Miyazaki, 2008). Collectively, these data demonstrate that the reduction of innate cell recruitment upon early treatment with rIL-27 has adverse effects on disease course and viral clearance; however, treatment at a later time-point, starting at the peak of viral load, did not impair viral clearance and antigen-specific responses while immunopathology and disease course were markedly improved.

### Detailed discussion of experimental examples

The tight regulation of both the induction and subsequent downregulation of proinflammatory cytokines is imperative in minimizing severe immunopathology during infection. One obvious example for this is influenza. Following infection with highly pathogenic strains of influenza viruses, a dramatic increase of leukocytic pulmonary infiltrates leads to the exaggerated production of pro-inflammatory cytokines that cause massive inflammation with increased mortality (La Gruta et al., 2007). Therefore, understanding the regulatory pathways during infection not only sheds light on the mechanisms controlling the delicate balance of efficient viral clearance and disastrous immunopathology, but also reveals potential therapeutic targets. The present invention therefore relates to IL-27 as a therapeutic candidate, as IL-27 plays a significant role in physiologically dampening inflammatory reactions while having little impact on virus elimination when applied at the later stages of influenza infection.

Initial experiments with IL-27Rα suggested an immunostimulatory role of IL-27, as signalling through its receptor mediated the upregulation of IL-12Rβ2 and IFN-y as well as induction of T-bet expression in CD4+ T cells in vitro (Batten and Ghilardi, 2007). However, recent data from multiple models of bacterial and parasitic infection revealed the suppressive function of IL-27 (Stumhofer and Hunter, 2008). When II-27ra-/- mice were infected with Mycobacterium tuberculosis, these mice displayed lower bacterial loads, but exhibited increased proinflammatory cytokines and later developed a more severe lung pathology (Holscher et al., 2005). Moreover, T. gondii-infected II-27ra-/- mice displayed unchanged IFN-y production relative to WT mice, but later developed an exaggerated CD4+ T cell response leading to decreased survival (Villarino et al., 2003). Similarly, II-27ra-/- mice infected with Leishmania donovani or Trypanosoma cruzi also exhibited a hyper-inflammatory response (Hamano et al., 2003; Rosas et al., 2006). In agreement with the suppressive role of IL-27, we found that mice lacking IL-27Ra exhibited accelerated mortality associated with increased lung injury after influenza virus challenge. Despite controlled viral loads, infected II-27ra-/- mice displayed increased neutrophil influx and exaggerated IFN-y and IL-17 production by CD4+ and CD8+ T cells, thus underlining a major role of IL-27 in regulating the generation of the inflammatory cytokines IFN-y, and IL-17 during influenza.

While multiple studies in mice lacking IFN-y failed to detect a major function of this cytokine in viral clearance (La Gruta et al., 2007), several lines of evidence suggest that IFN-y and TNF-a produced by CD8+ T cells play a significant role in mediating pulmonary damage during viral infections (Bruder et al., 2006; Hussell et al., 2001; Ostler et al., 2002; Peper and Van Campen, 1995; Wiley et al., 2001 Here we show that IL-27Ra signalling regulates IFNy production by CD8+ T cells after primary influenza challenge, which is also mirrored in vitro upon addition of IL-27 to cultures of plate-bound activated CD8+ T cells under Tc1 polarizing conditions. In contradiction to the observations by the Mohrs group (Mayer et al., 2008), our results strongly support the contention that IL-27 exerts a suppressive effect on IFN-y production by CD8+ T cells during influenza.

As shown here and in a number of infection models, IL-27 promotes IL-10 secretion in CD4+ T cells (Anderson et al., 2009; Awasthi et al., 2007; Fitzgerald et al., 2007; Stumhofer et al., 2006). However, in contrast to previously published observations (Sun et al., 2011), we observed no decrease in numbers of IL-10+IFN-y+ double-producing CD8+ T cells in the infected respiratory tract of II-27ra-/- mice. It is possible that the differential impact of IL-27 is due to the differences in mouse stains used to determine the function of IL-27. The use of II27ra-/- mice allowed us to abolish IL-27/IL-27Ra signalling completely, whereas the other group used Ebi3-/- mice, which could not exclude possible effects mediated solely by the IL-27p28 subunit.

It was subsequently examined whether misregulated IFN-y response in infected II-27ra-/- mice is solely due to diminished IL-10 production. We found that infected II-10-/- mice, unlike infected II27ra-/- mice, did not display an increase in IFN-y production by CD4+ and CD8+ T cells. Similar results were obtained upon blockade of the IL-10 receptor with a monoclonal antibody. Thus, IFN-y suppression in both CD4+ and CD8+ T cells is a result of direct signaling via the IL-27Rα and not indirectly through IL-10-mediated regulation. These observations were further verified in vitro by culturing fully activated IFN-y+CD8+ (Tc1) cells, whose production of IFN-y and IL-2 could be suppressed by IL-27, independent from the presence of IL-10. Interestingly, addition of IL-27 to cultures of fully activated CD4+ T cells results in STAT3 phosphorylation and dampened IFN-y production (Yoshimura et al., 2006), whereas in naive CD4+ T cells, IL-27 induces STAT1 and STAT3 phosphorylation leading to increased IFN-y production (Takeda et al., 2003; Yoshimura et al., 2006). As the levels of II-27ra expression were higher in antigen-experienced cells relative to naive T cells (Villarino et al., 2005), it is possible that the suppressive function of IL-27 might depend on the activation status of a T cell.

Recent studies in parasite infection or autoimmune models have highlighted the role of IL-27 in suppressing IL-17 responses (Batten et al., 2006; Rajaiah et al., 2010; Stumhofer et al., 2006). IL-27 was shown to regulate IL-17 secretion in CD4+ T cells through suppression of RORyt and osteopontin (Murugaiyan et al., 2010; Stumhofer et al., 2007). The present invention demonstrates that lack of IL-27Rα signalling during influenza leads to a dramatically increased IL-17 response by CD4+ and CD8+ T cells. In contrast to the direct suppression of IFN-y by IL-27, suppression of IL-17 appears largely to be mediated by IL-27-induced IL-10. In accordance with previous reports (McKinstry et al., 2009), the use of II-10-/- mice or blockade of IL-10R by an antibody during influenza resulted in elevated numbers of IL-17+CD4+ T cells, similar to that observed in II-27ra-/- mice. IL-17 contributes to acute lung injury during influenza virus infection, partly due to its ability to mediate neutrophil recruitment (Crowe et al., 2009; Hamada et al., 2009). It is therefore conceivable that an increase in IL-17, together with the exaggerated production of IFN-y and TNF-α, by CD4+ and CD8+ T cells in the respiratory tract of II-27ra-/- mice result in elevated numbers of neutrophils in the lungs, thus contributing to tissue damage. Indeed, influenza virus infected Ebi3-/- mice have increased neutrophil influx in the respiratory tract relative to WT mice (Sun et al., 2011).

The present invention demonstrates that the expression of the IL-27p28 subunit in the influenza virus-infected respiratory tract peaks at the later phase of infection when viral titers are already declining, which is consistent with the suggested role of IL-27 in limiting the immune response. Interferons can elicit IL-27 production (Hibbert et al., 2003; Remoli et al., 2007) and the II-27p28 gene promoter contains an IFN-stimulated response element region (ISRE), which becomes activated through IRF1 (Liu et al., 2007; Pirhonen et al., 2007). In this way, the expression of IL-27 will be turned on by the inflammatory microenvironment and serves as a negative feedback mechanism, thereby dampening the immune response in the later phase when adaptive immunity is established and the risk of severe immunopathology comes to the fore.

The data in the examples of the present invention provide clear evidence that a primary function of IL-27 in acute virus infection is to mount a physiological limit for inflammatory responses that might result in fatal immunopathology. The application of recombinant IL-27 (rIL-27) is of significant value in situations in which exaggerated immunopathology, rather than virus elimination, becomes a critical issue for host survival. The invention demonstrates that absence of IL-27 signalling led to a worsened disease course through uncontrolled inflammation, while viral titres in IL-27ra-/- animals were not significantly different from those of WT animals. Systemic application of daily doses of rIL-27 from day 5 to day 10 p.i. strongly suppressed the influx of neutrophils, macrophages and, to a lesser degree, NK cells into the lung. This finding was associated with decreased lung damage and accelerated recovery of treated animals. Surprisingly, the number of influenza-specific inflammatory cytokine production by T cells was not significantly changed, supporting the hypothesis that IL-27 can directly regulate innate cell recruitment to the lungs independently from other IL-27 regulated cytokines, including IFN-y and TNF-a. We assume that while systemically applied rIL-27 predominantly modulates the recruitment of innate cells from circulation and bone marrow, intraperitoneal application of rIL-27 does not further increase the already elevated levels of this cytokine in the lungs. Thus, the adaptive arm of the immune response appeared unaffected by treatment with rIL-27. Most importantly, viral clearance was well controlled, if not enhanced during rIL-27 treatment. Although macrophage and neutrophil infiltration to the inflamed lung is essential for host protection after infection (Julkunen et al., 2000; La Gruta et al., 2007; Tate et al., 2011), the influx of large numbers of these cells can contribute to lethal lung damage by producing inflammatory cytokines (e.g IL-1β, TNF-a, tFN-a/β) which can ultimately result in the amplification of chemotactic and inflammatory signals (Shaw et al., 1992). We speculate that the remaining macrophages and neutrophils in the lungs after treatment was sufficient to control the infection, but minimally contributed to lung damage.

Previous attempts to treat immunopathology during influenza using unspecific immunosuppressants like corticosteroids were found to worsen the disease, while a combination of anti-viral therapy and anti-inflammatory non-steroidals inhibiting cyclooxygenases improved survival in mice (Zheng et al., 2008). The application of a more selective immunomodulator such as IL-27 provides novel options for future treatment.

The biological importance of the delayed expression of IL-27 in influenza is emphasized when infected mice were treated with rIL-27 in the early phase. Early rIL-27 treatment resulted in suppressed neutrophil and macrophage influx in the lungs, leading to increased viral burden and more severe disease course in treated mice, although T cell responses remain unaffected. This finding re-inforces the role of innate cells in controlling the virus early during infection, as neutrophil and macrophage depletion led to increased viral titers and accelerated mortality during influenza (Herold et al., 2008; Tumpey et al., 2005).

In conclusion, the use of rIL-27 under a timed treatment regime shows surprisingly beneficial effects that are clinically applicable in cases of virus infection, for example cases in which severe immunopathology might lead to a fatal outcome.

### EXPERIMENTAL PROCEDURES

### Mice and infection

Wild-type (WT) BALB/c, C57BL/6 and Stat4-/- mice were purchased from Taconic Farms, Charles River, or The Jackson Laboratory. Strains of II-10-/- (a kind gift from Dr. W. Müller), Stat4-/- and II-12p40-/- mice were housed in specific pathogen-free facilitites in the Charite mouse facility, Berlin, Germany. II-27ra-/- mice (Yoshida et al., 2001) were bred and housed in the Department of Pathobiology at The University of Pennsylvania, Philadelphia, USA. Animal care and experiments were performed in accordance with the institutional guidelines of German Federal Law and local authorities of Berlin (LAGESO), or Animal Care and Use Committee of the University of Pennsylvania.

### Flow Cytometry

All FACS antibodies were purchased from BD Biosciences or ebioscience. Surface and intracellular cytokine staining (ICS) was performed as previously described (Hamada et al., 2009). Labeled cells were analyzed on a FACS Canto II (BD Bioscience). Data were analyzed using Flowjo analysis software (Treestar).

### Quantitative reverse-transcription PCR

RNA was extracted using RNeasy Mini Kit with oncolumn DNase digestion (Qiagen). cDNA was synthesized using SuperscriptII Reverse Transcriptase (Invitrogen) with random hexamers and oligo(dT) primers (Qiagen). Quantitative reverse-transcription PCR (qRT-PCR) was performed using Platinum SYBR Green qPCR SuperMix-UDG (Invitrogen) on a Stratagene MX3000 thermo cycler.

### In vitro activation of CD8+ T cells

Naive CD8+ T cells (CD8+CD62L+) were isolated from pooled splenocyte and lymph node samples using magnetic bead separation (Miltenyi). Purified CD8+ T cells were plated in 24- well plates and activated with plate-bound anti-CD3 (3µg/mL; clone 145-2C11) and antiCD28 (3 µg/mL; clone 37.51).

### In vivo treatment

Blockade of IL-10 signaling in vivo was performed as previously described (Sun et al., 2009). For recombinant IL-27 (rIL-27) treatment during infection, C57BL/6 mice were treated daily by i.p. injection of rIL-27 (200 ng; eBioscience) in PBS either from day 1 to day 7 p.i. (early treatment) or from day 5 to day 9 p.i. (late treatment).

### Histology, Immunohistochemistry and tissue preparation

For histological analysis, lung samples were fixed with 4% formaldehyde and embedded in paraffin. Sections were cut (2 µm), de-paraffinized and stained with hematoxylin and eosin (H&E).

### Statistical analysis

Data are means ± SD. Statistical tests used include Kaplan-Meier log-rank survival test, and Students t test. All P values > 0.05 are considered not to be significant.

### Mice and Infection

Influenza A/PR/8/34 virus was grown in the allantoic cavaties of 11-day-old embryonated chicken eggs or was purchased from Charles River. Mice were infected intranasally while under dexdomitor (0.5mg/mL, Pfizer) and anti-mepazole (5mg/mL, Pfizer) anesthesia, or with isofluran. For infection using low virus doses, wild-type BALB/c and Stat4-/- mice were infected with 12.5 PFU, while wild-type C57BL/6, II-10-/- and II-27ra-/- mice were infected with 25 PFU or 2500 egg infectious dose. In cases of infection with intermediate doses, 45 PFU of the virus was used. Infected mice were weighed daily and assessed for clinical symptoms of infection.

### Flow cytometry and intracellular cytokine detection

All FACS antibodies were purchased from BD Biosciences or eBioscience. Effector cells were identified according to the following surface markers: NK cells, CD3- CD49b+ (DX-5); monocytes, CD11b+MHCIIIo; neutrophils, Ly6-G+CD11 b+. The following monoclonal rat anti-mouse antibodies were used for staining cell surface markers: efluor®450-conjugated anti-CD4 (RM4-5), fluorescein-conjugated anti-Ly6- G (RB6-8C5), fluorescein-conjugated anti-CD49b (pan-NK), PerCP-Cy5.5-conjugated anti-CD11b (M1-70; all from eBioscience); PE-conjugated CD8a+ (53-6.7), PerCPconjugated anti-CD8+ (53-6.7), fluorescein or PE-conjugated anti-CD62L (16A/MEL14), PE-conjugated anti-CD3 (145-2C11; all from BD Pharmingen). When propidium iodide (PI) was used, it was added at 40 µg/ml to the cells immediately prior to cell acquisition. For intracellular cytokine staining, the following monoclonal rat antimouse antibodies were used: fluorescein isothiocyanate-conjugated anti-IFN-y (AN 18.17.24), PE or PE-Cy7-conjugated anti-IFN-y (XMG1.2) PE or allophycocyanin-conjugated anti-IL-10 (JESS-A6E3), PE-conjugated anti-IL-17A (TC11-18H10), PE-Cy7-conjugated anti-TNF-a (MP6-XT22; all from BD Pharmingen). Intracellular cytokine staining (ICS) was performed as previously described (Hamada et al., 2009). Briefly, enriched lymphocyte samples were restimulated with PMA/ionomycin for 4 hours or for 6 hours when using a combination of immunodominant influenza virus peptides (Anaspec), in the presence of Brefeldin A (10 µg/mL, Sigma). Supernatants of lung and BAL-cell restimulates were kept for cytokine measurement using ELISA. Cells were incubated with antiFcRy receptor antibody for 5 minutes prior to staining for extracellular markers. Extracellularly labeled cells were washed then fixed for 20 minutes using 2% paraformaldehyde. Prior to intacellular staining, the cells were permeabilized by 5 minutes incubation with 0.1 % saponin buffer. Cytokines were stained using fluorochrome-labeled monoclonal rat anti-mouse antibodies in 0.1 % saponin buffer for 20 minutes. After washing, the cells were resuspended in PBS/BSA then analyzed on a FACS Canto II (BD Biosciences). Data were analyzed using Flowjo analysis software (TreeStar).

### Influenza virus peptide sequences

For antigen-specific CD8+ T cell restimulation in vitro, the following influenza virus peptides are used: NP366-374 (ASNEMNDAM), PA224-233 (SSLENFRAYV), NS2114-121 (RTFSFQLI). For antigen-specific CD4+ T cell restimulation in vitro, the following influenza virus peptides are used: NP261-275 (RSALILRGSVAHKSC) and HNT126-138 (HNTNGVTAACHSE). All peptides were obtained from Anaspec.

### Quantitative reverse-transcription PCR primers

The following primers were used for qRT-PCR analysis:
HPRT forward: ATCATTATGCCGAGGATTTGGAA; HPRT reverse:
   TTGAGCACACACAGAGGGCC; 18s forward: GATCCATTGGAGGGCAAGTCT;18s reverse: GCAGCAACTTTAATATACGCTATTGC; EBI3 forward:
GGCTGAGCGAATCATCAAG; EBI3 reverse: CTGTGAGGTCCTGAGCTGAC;IL27p28 forward: CTGGTACAAGCTGGTTCCTG; IL-27p28reverse:
   TCAGAGTCAGAGAGGTGATGC; IL-10 forward: GCACTACCAAAGCCACAAG; IL-10 reverse: TGTTTGAAAGAAAGTCTTCACCTG; PA forward: CGGTCCAAATTCCTGCTGA; PA reverse: CATTGGGTTCCTTCCATCCA (Crowe et al., 2009; McKinstry et al., 2009).

### Preparation of Tc1 cultures

Purified naïve CD8+ T cells were supplemented with recombinant mouse IL-12 (5 ng/mL; eBioscience), IFN-y (20 ng/mL; eBioscience), and IL-2 (10 ng/mL; R&D Systems) plus anti-mouse IL-4 antibody (5 µg/mL; clone 11B11) in complete RPMI media (RPMI 1640 supplemented with 10% FCS and antibiotics) for 3 days. After 3 days in culture, Tc1 cells were transferred to a new plate without anti-CD3 and antiCD28 and supplemented with fresh medium plus rIL-2 and cultured for 2 days for a total of 5 days in culture. In cases where recombinant mouse IL-27 (rIL-27) was added or IL-10 receptor was blocked, the media were supplemented with rIL-27 (50 ng/mL; eBioscience) and/or anti-IL-10 receptor blocking antibody (αIL-10R) (40 µg/mL; 1B1- 2). In some experiments, cells were labeled with CFSE (Sigma) and cultured under the same conditions as mentioned above. For detections of cytokines in vitro, Tc1 cells were washed and restimulated with PMA/ionomycin, in the presence of Brefeldin-A (10 µg/mL; Sigma) for 4 hours prior to intracellular staining. For detailed description of intracellular staining, see intracellular cytokine detection.

### Bronchoalveolar lavage fluid and tissue preparation

Bronchoalveolar lavage (BAL) was performed by flushing the airways three times with 1 mL sterile PBS. Cells were spun down and BAL fluid was collected for ELISA or bicinchoninic acid assay (BCA). Single cell suspensions from lungs, lymph nodes and spleens were obtained by mashing through a 70 µm cell strainer (BD Falcon). Erythrocytes were lysed using an erylysis buffer (Sigma), then washed with complete RPMI media. To enrich for lung lymphocytes, cells were resuspended in a percoll gradient 40:70 (vol:vol), then centrifuged for 30 minutes at 1800 rpm. Lymphocytic cells at the interface were collected and washed twice.

### Assessment of lung injury and ELISA

Total protein in the BAL fluid was measured using the bicinchoninic protein assay (BCA) kit (Pierce Chemical). BCA was performed in 96-well plates according to the manufacturer's instructions and measured in a Spectra Max 190 plate reader. To determine cytokine concentration using ELISA, lung homogenates and BAL fluid were analyzed using the ELISA Duo-set kit for IL-10 or IFN-y (BD Biosciences) or IL-27 Ready-SET-go kit (eBiosciences). Absorbance was read using Spectra Max 190 plate reader and analysis was performed using the SoftMax software.

### Blocking IL-10 receptor in vivo

Blockade of IL-10 signaling in vivo was achieved by administration of an anti-IL-10 receptor-specific monoclonal antibody (αIL-10R; clone 1B1-2) or isotype control to C57BL/6 mice on day 3 (i.p., 1 mg in 200 µL PBS), day 4 (i.n., 0.15 mg in 30 µL PBS) and day 6 (i.p., 1 mg in 200 µL PBS) after a low dose influenza virus infection (Sun et al., 2009).

### Immunohistochemistry and histolopathological scoring

Immunochemistry staining was performed by subjecting lung sections to epitope retrieval followed by incubation with primary antibodies directed against CD3 (Dako) or MPO7 (Dako) for 30 minutes. Detection was performed using biotinylated antirabbit secondary antibody (Dianova) followed by streptavidin-AP kit (Dako). Alkaline phosphatase (AP) was revealed by Fast Red as chromogen. Negative controls were performed by omitting the primary antibodies. Images were acquired using an Axiolmager Z1 microscope equipped with a charge-coupled device (CCD) camera (AxioCam MRm) and processed with AxioVision software (all purchased from Carl Zeiss Microlmaging, Inc.). H&E stained lung sections were scored in a blinded manner as follows: (0) normal, (1) minor perivascular inflammation around large blood vessels, (2) moderate perivascular and peribronchial inflammation, (3) increased perivascular and peribronchial inflammation, (4) severe formation of perivascular, peribronchial, and interstitial inflammation.

### REFERENCES

Amadi-Obi, A., Yu, C.R., Liu, X., Mahdi, R.M., Clarke, G.L., Nussenblatt, R.B., Gery, I., Lee, Y.S., and Egwuagu, C.E. (2007). TH17 cells contribute to uveitis and scleritis and are expanded by IL-2 and inhibited by IL-27/STAT1. Nat Med 13, 711-718.
Anderson, C.F., Stumhofer, J.S., Hunter, C.A., and Sacks, D. (2009). IL-27 regulates IL-10 and IL-17 from CD4+ cells in nonhealing Leishmania major infection. J Immunol 183, 4619-4627.
Awasthi, A., Carrier, Y., Peron, J.P., Bettelli, E., Kamanaka, M., Flavell, R.A., Kuchroo, V.K., Oukka, M., and Weiner, H.L. (2007). A dominant function for interleukin 27 in generating interleukin 10- producing anti-inflammatory T cells. Nat Immunol 8, 1380-1389.
Batten, M., and Ghilardi, N. (2007). The biology and therapeutic potential of interleukin 27. J Mol Med (Berl) 85, 661-672.
Batten, M., Li, J., Yi, S., Kljavin, N.M., Danilenko, D.M., Lucas, S., Lee, J., de Sauvage, F.J., and Ghilardi, N. (2006). Interleukin 27 limits autoimmune encephalomyelitis by suppressing the development of interleukin 17-producing T cells. Nat Immunol 7, 929-936.
Bruder, D., Srikiatkhachorn, A., and Enelow, R.I. (2006). Cellular immunity and lung injury in respiratory virus infection. Viral Immunol 19, 147-155.
Crowe, C.R., Chen, K., Pociask, D.A., Alcorn, J.F., Krivich, C., Enelow, R.I., Ross, T.M., Witztum, J.L., and Kolls, J.K. (2009). Critical role of IL-17RA in immunopathology of influenza infection. J Immunol 183, 5301-5310.
Dong, J., Ivascu, C., Chang, H.D., Wu, P., Angeli, R., Maggi, L., Eckhardt, F., Tykocinski, L., Haefliger, C., Mowes, B., et al. (2007). IL-10 is excluded from the functional cytokine memory of human CD4+ memory T lymphocytes. J Immunol 179, 2389-2396.
Findlay, E.G., Greig, R., Stumhofer, J.S., Hafalla, J.C., de Souza, J.B., Saris, C.J., Hunter, C.A., Riley, E.M., and Couper, K.N. (2010). Essential role for IL-27 receptor signaling in prevention of Th1- mediated immunopathology during malaria infection. J Immunol 185, 2482-2492.
Fitzgerald, D.C., Zhang, G.X., El-Behi, M., Fonseca-Kelly, Z., Li, H., Yu, S., Saris, C.J., Gran, B., Ciric, B., and Rostami, A. (2007). Suppression of autoimmune inflammation of the central nervous system by interleukin 10 secreted by interleukin 27-stimulated T cells. Nat Immunol 8, 1372-1379.
Hamada, H., Garcia-Hernandez Mde, L., Reome, J.B., Misra, S.K., Strutt, T.M., McKinstry, K.K., Cooper, A.M., Swain, S.L., and Dutton, R.W. (2009). Tc17, a unique subset of CD8 T cells that can protect against lethal influenza challenge. J Immunol 182, 3469-3481. Hamano, S., Himeno, K., Miyazaki, Y., Ishii, K., Yamanaka, A., Takeda, A., Zhang, M., Hisaeda, H., Mak, T.W., Yoshimura, A., and Yoshida, H. (2003). WSX-1 is required for resistance to Trypanosoma cruzi infection by regulation of proinflammatory cytokine production. Immunity 19, 657-667.
Herold, S., Steinmueller, M., von Wulffen, W., Cakarova, L., Pinto, R., Pleschka, S., Mack, M., Kuziel, W.A., Corazza, N., Brunner, T., et al. (2008). Lung epithelial apoptosis in influenza virus pneumonia: the role of macrophage-expressed TNF-related apoptosis-inducing ligand. J Exp Med 205, 3065-3077.
Hibbert, L., Pflanz, S., De Waal Malefyt, R., and Kastelein, R.A. (2003). IL-27 and IFN-alpha signal via Stat1 and Stat3 and induce T-Bet and IL-12Rbeta2 in naive T cells. J Interferon Cytokine Res 23, 513-522.
Holscher, C., Holscher, A., Ruckerl, D., Yoshimoto, T., Yoshida, H., Mak, T., Saris, C., and Ehlers, S. (2005). The IL-27 receptor chain WSX-1 differentially regulates antibacterial immunity and survival during experimental tuberculosis. J Immunol 174, 3534-3544.
Hunter, C.A. (2005). New IL-12-family members: IL-23 and IL-27, cytokines with divergent functions. Nat Rev Immunol 5, 521-531.
Hussell, T., Pennycook, A., and Openshaw, P.J. (2001). Inhibition of tumor necrosis factor reduces the severity of virus-specific lung immunopathology. Eur J Immunol 31, 2566-2573.
Julkunen, I., Melen, K., Nyqvist, M., Pirhonen, J., Sareneva, T., and Matikainen, S. (2000). Inflammatory responses in influenza A virus infection. Vaccine 19 Suppl 1, S32-37.
La Gruta, N.L., Kedzierska, K., Stambas, J., and Doherty, P.C. (2007). A question of self-preservation: immunopathology in influenza virus infection. Immunol Cell Biol 85, 85-92.
Li, J.P., Wu, H., Xing, W., Yang, S.G., Lu, S.H., Du, W.T., Yu, J.X., Chen, F., Zhang, L., and Han, Z.C. (2010). Interleukin-27 as a negative regulator of human neutrophil function. Scand J Immunol 72, 284-292.
Liu, J., Guan, X., and Ma, X. (2007). Regulation of IL-27 p28 gene expression in macrophages through MyD88- and interferon-gamma-mediated pathways. J Exp Med 204, 141-152.
Lucas, S., Ghilardi, N., Li, J., and de Sauvage, F.J. (2003). IL-27 regulates IL-12 responsiveness of naive CD4+ T cells through Stat1-dependent and -independent mechanisms. Proc Natl Acad Sci U S A 100, 15047-15052.
Mayer, K.D., Mohrs, K., Reiley, W., Wittmer, S., Kohlmeier, J.E., Pearl, J.E., Cooper, A.M., Johnson, L.L., Woodland, D.L., and Mohrs, M. (2008). Cutting edge: T-bet and IL-27R are critical for in vivo IFN-gamma production by CD8 T cells during infection. J Immunol 180, 693-697.
McKinstry, K.K., Strutt, T.M., Buck, A., Curtis, J.D., Dibble, J.P., Huston, G., Tighe, M., Hamada, H., Sell, S., Dutton, R.W., and Swain, S.L. (2009). IL-10 deficiency unleashes an influenza-specific Th17 response and enhances survival against high-dose challenge. J Immunol 182, 7353-7363.
Murugaiyan, G., Mittal, A., and Weiner, H.L. (2010). Identification of an IL-27/osteopontin axis in dendritic cells and its modulation by IFN-gamma limits IL-17-mediated autoimmune inflammation. Proc Natl Acad Sci U S A 107, 11495-11500.
Ostler, T., Davidson, W., and Ehl, S. (2002). Virus clearance and immunopathology by CD8(+) T cells during infection with respiratory syncytial virus are mediated by IFN-gamma. Eur J Immunol 32, 2117-2123.
Owaki, T., Asakawa, M., Morishima, N., Hata, K., Fukai, F., Matsui, M., Mizuguchi, J., and Yoshimoto, T. (2005). A role for IL-27 in early regulation of Th1 differentiation. J Immunol 175, 2191-2200.
Passos, S.T., Silver, J.S., O'Hara, A.C., Sehy, D., Stumhofer, J.S., and Hunter, C.A. (2010). IL-6 promotes NK cell production of IL-17 during toxoplasmosis. J Immunol 184, 1776-1783.
Peiris, J.S., Hui, K.P., and Yen, H.L. (2010). Host response to influenza virus: protection versus immunopathology. Curr Opin Immunol 22, 475-481.
Peper, R.L., and Van Campen, H. (1995). Tumor necrosis factor as a mediator of inflammation in influenza A viral pneumonia. Microb Pathog 19, 175-183.
Pflanz, S., Timans, J.C., Cheung, J., Rosales, R., Kanzler, H., Gilbert, J., Hibbert, L., Churakova, T., Travis, M., Vaisberg, E., et al. (2002). IL-27, a heterodimeric cytokine composed of EBI3 and p28 protein, induces proliferation of naive CD4(+) T cells. Immunity 16, 779-790.
Pirhonen, J., Siren, J., Julkunen, I., and Matikainen, S. (2007). IFN-alpha regulates Toll-like receptormediated IL-27 gene expression in human macrophages. J Leukoc Biol 82, 1185-1192.
Rajaiah, R., Puttabyatappa, M., Polumuri, S.K., and Moudgil, K.D. (2010). Interleukin-27 and interferon-gamma are involved in regulation of autoimmune arthritis. J Biol Chem 286, 2817-2825.
Remoli, M.E., Gafa, V., Giacomini, E., Severa, M., Lande, R., and Coccia, E.M. (2007). IFN-beta modulates the response to TLR stimulation in human DC: involvement of IFN regulatory factor-1 (IRF-1) in IL-27 gene expression. Eur J Immunol 37, 3499-3508.
Rosas, L.E., Satoskar, A.A., Roth, K.M., Keiser, T.L., Barbi, J., Hunter, C., de Sauvage, F.J., and Satoskar, A.R. (2006). Interleukin-27R (WSX-1/T-cell cytokine receptor) gene-deficient mice display enhanced resistance to leishmania donovani infection but develop severe liver immunopathology. Am J Pathol 168, 158-169.
Saraiva, M., Christensen, J.R., Veldhoen, M., Murphy, T.L., Murphy, K.M., and O'Garra, A. (2009). Interleukin-10 production by Th1 cells requires interleukin-12-induced STAT4 transcription factor and ERK MAP kinase activation by high antigen dose. Immunity 31, 209-219.
Shaw, M.W., Arden, N.H., and Maassab, H.F. (1992). New aspects of influenza viruses. Clin Microbiol Rev 5, 74-92.
Stumhofer, J.S., and Hunter, C.A. (2008). Advances in understanding the anti-inflammatory properties of IL-27. Immunol Lett 117, 123-130.
Stumhofer, J.S., Laurence, A., Wilson, E.H., Huang, E., Tato, C.M., Johnson, L.M., Villarino, A.V., Huang, Q., Yoshimura, A., Sehy, D., et al. (2006). Interleukin 27 negatively regulates the development of interleukin 17-producing T helper cells during chronic inflammation of the central nervous system. Nat Immunol 7, 937-945.
Stumhofer, J.S., Silver, J., and Hunter, C.A. (2007). Negative regulation of Th17 responses. Semin Immunol 19, 394-399.
Sun, J., Dodd, H., Moser, E.K., Sharma, R., and Braciale, T.J. (2011). CD4+ T cell help and innatederived IL-27 induce Blimp-1-dependent IL-10 production by antiviral CTLs. Nat Immunol 12, 327- 334.
Sun, J., Madan, R., Karp, C.L., and Braciale, T.J. (2009). Effector T cells control lung inflammation during acute influenza virus infection by producing IL-10. Nat Med 15, 277-284.
Takeda, A., Hamano, S., Yamanaka, A., Hanada, T., Ishibashi, T., Mak, T.W., Yoshimura, A., and Yoshida, H. (2003). Cutting edge: role of IL-27/WSX-1 signaling for induction of T-bet through activation of STAT1 during initial Th1 commitment. J Immunol 170, 4886-4890.
Tate, M.D., loannidis, L.J., Croker, B., Brown, L.E., Brooks, A.G., and Reading, P.C. (2011). The role of neutrophils during mild and severe influenza virus infections of mice. PLoS One 6, e17618.
Tumpey, T.M., Basler, C.F., Aguilar, P.V., Zeng, H., Solorzano, A., Swayne, D.E., Cox, N.J., Katz, J.M., Taubenberger, J.K., Palese, P., and Garcia-Sastre, A. (2005). Characterization of the reconstructed 1918 Spanish influenza pandemic virus. Science 310, 77-80.
Villarino, A., Hibbert, L., Lieberman, L., Wilson, E., Mak, T., Yoshida, H., Kastelein, R.A., Saris, C., and Hunter, C.A. (2003). The IL-27R (WSX-1) is required to suppress T cell hyperactivity during infection. Immunity 19, 645-655.
Villarino, A.V., Larkin, J., 3rd, Saris, C.J., Caton, A.J., Lucas, S., Wong, T., de Sauvage, F.J., and Hunter, C.A. (2005). Positive and negative regulation of the IL-27 receptor during lymphoid cell activation. J Immunol 174, 7684-7691.
Villarino, A.V., Stumhofer, J.S., Saris, C.J., Kastelein, R.A., de Sauvage, F.J., and Hunter, C.A. (2006). IL-27 limits IL-2 production during Th1 differentiation. J Immunol 176, 237-247.
Wiley, J.A., Cerwenka, A., Harkema, J.R., Dutton, R.W., and Harmsen, A.G. (2001). Production of interferon-gamma by influenza hemagglutinin-specific CD8 effector T cells influences the development of pulmonary immunopathology. Am J Pathol 158, 119-130.
Wirtz, S., Tubbe, I., Galle, P.R., Schild, H.J., Birkenbach, M., Blumberg, R.S., and Neurath, M.F. (2006). Protection from lethal septic peritonitis by neutralizing the biological function of interleukin 27. J Exp Med 203, 1875-1881.
Yoshida, H., Hamano, S., Senaldi, G., Covey, T., Faggioni, R., Mu, S., Xia, M., Wakeham, A.C., Nishina, H., Potter, J., et al. (2001). WSX-1 is required for the initiation of Th1 responses and resistance to L. major infection. Immunity 15, 569-578.
Yoshida, H., and Miyazaki, Y. (2008). Regulation of immune responses by interleukin-27. Immunol Rev 226, 234-247.
Yoshimura, T., Takeda, A., Hamano, S., Miyazaki, Y., Kinjyo, I., Ishibashi, T., Yoshimura, A., and Yoshida, H. (2006). Two-sided roles of IL-27: induction of Th1 differentiation on naive CD4+ T cells versus suppression of proinflammatory cytokine production including IL-23-induced IL-17 on activated CD4+ T cells partially through STAT3-dependent mechanism. J Immunol 177, 5377-5385.
Zheng, B.J., Chan, K.W., Lin, Y.P., Zhao, G.Y., Chan, C., Zhang, H.J., Chen, H.L., Wong, S.S., Lau, S.K., Woo, P.C., et al. (2008). Delayed antiviral plus immunomodulator treatment still reduces mortality in mice infected by high inoculum of influenza A/H5N1 virus. Proc Natl Acad Sci U S A 105, 8091-8096.

## Claims

1. Interleukin-27-modulator for the treatment of a pathological immune response associated with acute lung disease.

2. IL-27 modulator according to the preceding claim, **characterised in that** the modulator is
a. an IL-27 agonist, such as
i. IL-27 protein, preferably recombinant IL-27, comprising of complete recombinant cytokine IL-27 or a fragment thereof that maintains activity, for example one of the IL-27 chains, p28 or EBI3,
ii. a nucleic acid that encodes IL-27 according to any one of the preceding claims, or encodes an IL-27 fragment, that after administration and expression of said nucleic acids leads to the local production of IL-27 protein, or
iii. a bacterial or viral host organism that comprises a nucleic acid that encodes IL-27 or a functional fragment thereof, for example one of the IL-27 chains, p28 or EBI3, or
b. an IL-27 antagonist, such as
i. antibodies that bind to IL27, to its subunits p28 and/or EBI3, antibodies directed against the IL-27 receptor or its subunits, IL27-receptor fusion proteins competing for soluble IL-27, or
ii. antagonistic compounds blocking the receptors including inactive or inactivated variants of IL-27 or its subunits, or
iii. a nucleic acid that encodes IL27-receptor fusion proteins or antagonistic compounds according to any one of the preceding claims, that after administration and expression of said nucleic acids leads to the local production of an IL-27 antagonist.

3. IL-27 modulator according to any one of the preceding claims, **characterised in that** the acute lung disease is a non-allergic and/or non-chronic lung disease.

4. IL-27 agonist according to any one of the preceding claims, **characterised in that** the pathological immune response is an inflammatory immune response, such as pneumonia.

5. IL-27 agonist according to any one of the preceding claims, **characterised in that** the inflammation is caused by an infectious agent, such as viral, bacterial, fungal or parasitic infectious agents and may include concurrent infections by one or more of these agents.

6. IL-27 agonist according to the preceding claim, **characterised in that** the infectious agent is
- an infection by influenza-, rhino-, corona-, herpes (such as cytomegalovirus (CMV) or varizella zoster virus (VZV)), respiratory syncytial or other viruses,
- an infection by Streptococcus, Staphylococcus, Pseudomonas, Haemophilus, Chlamydophila or other bacteria including Pneumocystis, such as Pneumocystis jiroveci pneumoniam, and/or
- an infection by Aspergillus, Candida or other mycosis.

7. IL-27 agonist according to claims 1 to 4, **characterised in that** the inflammation is caused by a non-infectious agent or treatment, including but not restricted to mechanical ventilation, trauma, transfusion related, disseminated coagulopathy, acid aspiration, idiopathic (Haman-Rich Syndrome) and any other sterile causes of ARDS (acute respiratory distress syndrome).

8. IL-27 agonist according to the preceding claim, **characterised in that** the pathological immune response in the lung is an acute respiratory distress syndrome (ARDS or ALI).

9. IL-27 agonist according to any one of the preceding claims, **characterised in that** administration is carried out at an optimal and safe time point defined by a diagnostic tests for immunological parameters.

10. IL-27 agonist according to any one of the preceding claims, **characterised in that** treatment with IL-27 agonist occurs at a defined, preferably delayed, stage of infection, whereby the viral and/or bacterial load during progression of the infection has gone beyond the exponential phase of proliferation post infection, for example when the peak viral and/or bacterial load has been attained, or preferably has passed.

11. IL-27 agonist according to any one of the preceding claims, **characterised in that** the stage of infection during progression of the infection and/or the stage of pathological immune response is determined by measurement of
- circulating virus and/or bacteria, or viral and/or bacterial components, such as protein, RNA and/or DNA.
- specific immune indicators, such as markers of acute inflammation, for example IL-6, IL-8 or C-reactive protein (CRP),
- markers for infection, such as Procalcitonin or Lipopolysaccharide (LPS) binding protein (LBP), and/or
- antibodies, preferably specific antibodies, more preferably antibodies specific to infection-related virus and/or bacteria, or viral and/or bacterial components, and/or T cells in the patient.

12. IL-27 agonist according to any one of the preceding claims, **characterised in that** the treatment comprises of daily administration of IL-27 agonist for a duration of 2 to 14 days, preferably 4 to 12 days, more preferably 5 to 10 days.

13. IL-27 antagonist according to claims 1 to 3 for the treatment of a pathological immune response, whereby the immune response is immune response suppression.

14. IL-27 antagonist according to the preceding claim, **characterised in that** the suppressed immune response occurs during or subsequent to influenza infection, preferably in case of multiple infections by bacterial and/or viral agents, for example double infections of influenza and pneumococcus.

15. IL-27 antagonist according to any one of the preceding claims, **characterised in that** the occurrence of a immunodepressed state, as a indication for treatment, is determined by diagnostic tests measuring appropriate markers of an immunodepression including but not restricted to IL-27 levels in blood or other body fluids, IL-10, HLA-DR expression on monocytes, LPS-induced cytokine (e.g. TNF) in monocytes, CD64 expression in granulocytes, or any other parameter defining a condition of endogenous suppression of the immune system.

16. Pharmaceutical composition comprising the IL-27 modulator of claims 1 to 3, the IL-27 agonist of claims 4 to 12, the IL-27 antagonist of claims 13 to 15, with a pharmaceutically acceptable carrier substance.
